# EUROPEAN PATENT APPLICATION

(11) **EP 2 123 646 A1**
(43) Date of publication of application: **25.11.2009**
(21) Application number: 08720303.0
(22) Date of filing: 28.02.2008
(51) Int. Cl.: C07D 313/04

(54) **METHOD FOR PRODUCING ESTER OR LACTONE**

(30) Priority: 08.03.2007 JP 2007059229
(71) Applicant: Daicel Chemical Industries, Ltd., Kita-ku, Osaka-shi Osaka 530-0001 (JP)
(72) Inventor: ISHII, Yasutaka, Takatsuki-shi Osaka 569-1112 (JP); IWAHAMA, Takahiro, Himeji-shi Hyogo 671-1283 (JP)
(74) Representative: Grünecker, Kinkeldey, Stockmair & Schwanhäusser Anwaltssozietät
(86) International application number: PCT/JP2008/000389
(87) International publication number: WO 2008/108072

(57) **Abstract**

Disclosed is a method for producing an ester or lactone in which a secondary alcohol represented by following Formula (1) (wherein R^{a} and R^{b} each represent an organic group, or R^{a} and R^{b} may be combined to form a ring together with the adjacent carbon atom) is oxidized by molecular oxygen in the presence of a nitrogen-containing cyclic compound containing a structure represented by following Formula (I) [wherein X represents an oxygen atom or an -OR group (wherein R represents a hydrogen atom or hydroxyl-protecting group)] as a constituent of its ring, and at least one of a fluorine-containing alcohol and a fluorinated sulfonic acid, and thereby yields a compound represented by following Formula (2):

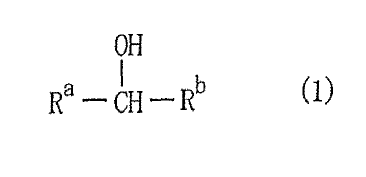

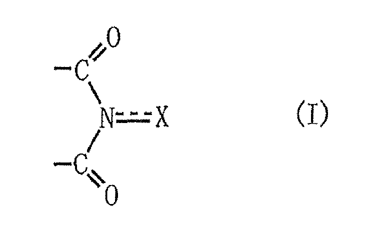

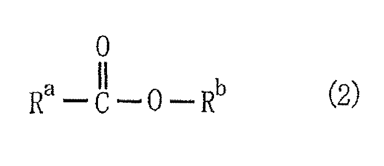

## Description

### Technical Field

The present invention relates to methods for producing esters or lactones. More specifically it relates to methods for producing corresponding esters or lactones through oxidation of secondary alcohols and ketones by molecular oxygen.

### Background Art

Esters or lactones are important compounds as pharmaceuticals, flavors, dyestuffs, intermediates for organic syntheses, and raw materials for polymeric resins. A known method for obtaining an ester or lactone is a method for producing an ester or lactone through so-called a Baeyer-Villiger rearrangement (oxidation), in which a chain or cyclic ketone is reacted with a peracid such as perbenzoic acid, peracetic acid, or trifuloroperacetic acid. The peracid is, however, unstable and should be handled with extreme caution. In addition, an equivalent amount of a carboxylic acid is by-produced in the reaction using the peracid.

Another known method for producing an ester or lactone is a method of oxidizing a secondary alcohol by molecular oxygen in the presence of an imide compound, and treating the oxidation product with an acid to give a corresponding ester or lactone (see Patent Document 1). This method, however, cannot be said to be sufficiently satisfactory as an industrial method, because the selectivity of the target ester or lactone is low. Yet another known method is a method of oxidizing a secondary alcohol by molecular oxygen in the presence of a nitrogen-containing cyclic compound, and treating the oxidation product with a compound containing an element belonging to the fourth to sixth periods of Group 16 of the Periodic Table, to give a corresponding ester or lactone (see Patent Document 2). However, this method is also not satisfactory in the point typically of easiness in separation and purification of the target compound.

Patent Document 1: PCT International Publication Number WO 99/50204
Patent Document 2: Japanese Unexamined Patent Application Publication (JP-A) No. 2004-256483

### Disclosure of Invention

### Problems to be Solved by the Invention

Accordingly, an object of the present invention is to provide a method for industrially efficiently producing a corresponding ester or lactone from a secondary alcohol or an oxidation product thereof (peroxide) in a high selectivity through a simple and easy operation.

Another object of the present invention is to provide a method for industrially efficiently producing a corresponding ester or lactone from a ketone in a high selectivity through a simple and easy operation.

### Means for Solving the Problems

After intensive investigations to achieve the objects, the present inventors have found that at least one of a fluorine-containing alcohol and a fluorinated sulfonic acid, if used in combination with a nitrogen-containing cyclic compound catalyst such as a cyclic imide compound in an oxidation reaction of a secondary alcohol, helps a so-called Baeyer-Villiger reaction to proceed more smoothly to thereby improve the selectivity for a corresponding ester or lactone; and that such fluorine-containing alcohol and/or fluorinated sulfonic acid also helps to immediately decompose a peroxide of specific structure, as an oxidation product of the secondary alcohol (dimerized peroxide of the secondary alcohol), to give an ester or lactone corresponding to the secondary alcohol. After further investigations, they have found that a reaction [a so-called Baeyer-Villiger reaction in the cases of (i), (ii), (iv), and (v) below] proceeds under mild conditions to give a corresponding ester or lactone in a high selectivity to thereby produce the ester or lactone industrially efficiently, (i) by oxidizing a secondary alcohol by molecular oxygen in the presence of a specific nitrogen-containing cyclic compound catalyst and at least one of a fluorine-containing alcohol and a fluorinated sulfonic acid; (ii) by oxidizing a secondary alcohol by molecular oxygen in the presence of a specific nitrogen-containing cyclic compound catalyst, and treating the oxidation product with at least one of a fluorine-containing alcohol and a fluorinated sulfonic acid; (iii) by treating a peroxide having a specific structure, as an oxidation product of a secondary alcohol, with at least one of a fluorine-containing alcohol and a fluorinated sulfonic acid; (iv) by oxidizing a ketone by molecular oxygen in the presence of a secondary alcohol, a specific nitrogen-containing cyclic compound catalyst, and at least one of a fluorine-containing alcohol and a fluorinated sulfonic acid; or (v) by oxidizing a ketone by molecular oxygen in the presence of a specific nitrogen-containing cyclic compound catalyst, and treating the oxidation product with at least one of a fluorine-containing alcohol and a fluorinated sulfonic acid. The present invention has been made based on these findings.

Specifically, the present invention provides a method for producing an ester or lactone, which method includes the step of oxidizing a secondary alcohol by molecular oxygen in the presence of a nitrogen-containing cyclic compound and at least one of a fluorine-containing alcohol and a fluorinated sulfonic acid, the secondary alcohol represented by following Formula (1): 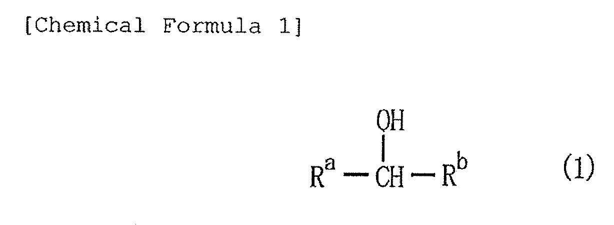 (wherein R^{a} and R^{b} are the same as or different from each other and each represent an organic group having a carbon atom at a bonding site with the adjacent carbon atom, or R^{a} and R^{b} may be combined to form a ring together with the adjacent carbon atom),
the nitrogen-containing cyclic compound containing, as a constituent of its ring, a skeleton represented by following Formula (I): 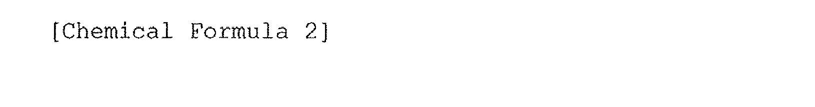 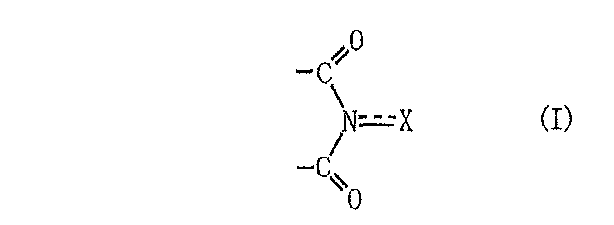 [wherein X represents an oxygen atom or an -OR group where R represents a hydrogen atom or a hydroxyl-protecting group], to give a compound represented by following Formula (2): 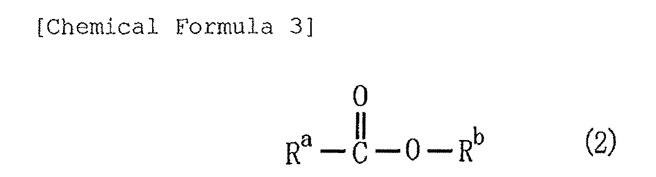 (wherein R^{a} and R^{b} are the same as or different from each other and each represent an organic group having a carbon atom at a bonding site with the adjacent carbonyl-carbon atom or oxygen atom, or R^{a} and R^{b} may be combined to form a ring together with the adjacent carbonyl-carbon atom and oxygen atom). This method is hereinafter also referred to as "first production method of esters or lactones".

In this production method, the secondary alcohol represented by Formula (1) may be oxidized in the presence of a ketone.

The present invention further provides a method for producing an ester or lactone, which method includes the steps of: oxidizing a secondary alcohol by molecular oxygen in the presence of a nitrogen-containing cyclic compound, the secondary alcohol represented by following Formula (1): 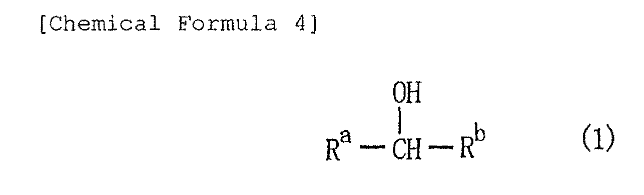 (wherein R^{a} and R^{b} are the same as or different from each other and each represent an organic group having a carbon atom at a bonding site with the adjacent carbon atom, or R^{a} and R^{b} may be combined to form a ring together with the adjacent carbon atom),
the nitrogen-containing cyclic compound containing, as a constituent of its ring, a skeleton represented by following Formula (I): 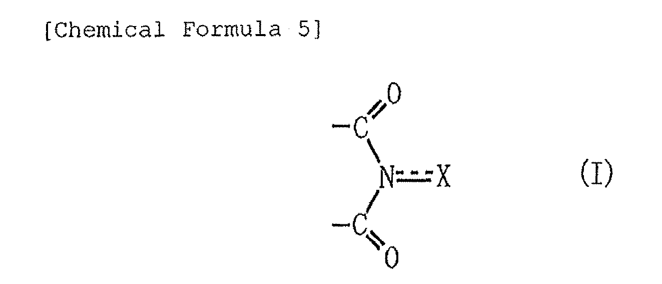 (wherein X represents an oxygen atom or an -OR group where R represents a hydrogen atom or a hydroxyl-protecting group); and treating the oxidation product with at least one of a fluorine-containing alcohol and a fluorinated sulfonic acid, to give a compound represented by following Formula (2): 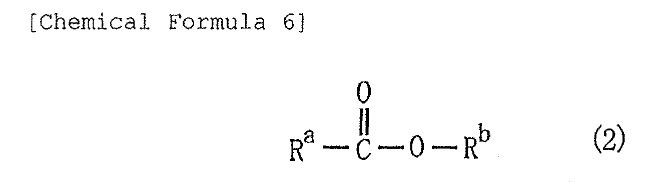 (wherein R^{a} and R^{b} are the same as or different from each other and each represent an organic group having a carbon atom at a bonding site with the adjacent carbonyl-carbon atom or oxygen atom, or R^{a} and R^{b} may be combined to form a ring together with the adjacent carbonyl-carbon atom and oxygen atom). This method is hereinafter also referred to as "second production method of esters or lactones".

In this production method, the secondary alcohol represented by Formula (1) may be oxidized in the presence of a ketone.

The present invention further provides a method for producing an ester or lactone, which method includes the step of treating a peroxide with at least one of a fluorine-containing alcohol and a fluorinated sulfonic acid, the peroxide represented by following Formula (3a) and/or (3b): 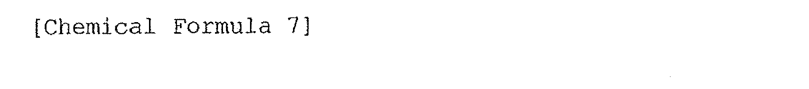 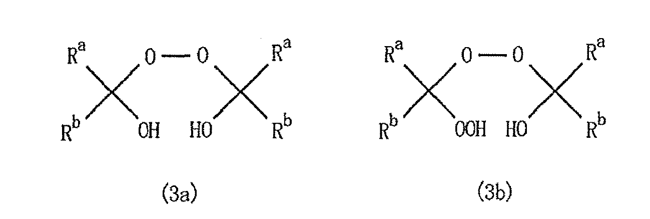 (wherein R^{a}s and R^{b}s are the same as or different from one another and each represent an organic group having a carbon atom at a bonding site with the adjacent carbon atom, or R^{a} and R^{b} may be combined to form a ring together with the adjacent carbon atom),
to give a compound represented by following Formula (2): 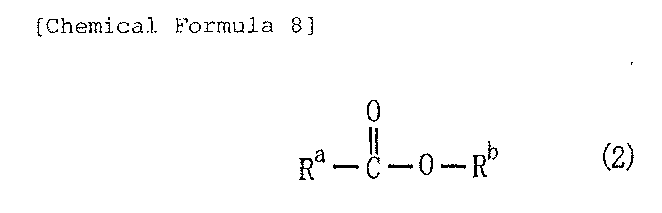 (wherein R^{a} and R^{b} are the same as or different from each other and each represent an organic group having a carbon atom at a bonding site with the adjacent carbonyl-carbon atom or oxygen atom, or R^{a} and R^{b} may be combined to form a ring together with the adjacent carbonyl-carbon atom and oxygen atom). This method will be also referred to as "third production method of esters or lactones".

In addition, the present invention provides a method for producing an ester or lactone, which method includes the step of oxidizing a ketone by molecular oxygen in the presence of a secondary alcohol, a nitrogen-containing cyclic compound, and at least one of a fluorine-containing alcohol and a fluorinated sulfonic acid, the ketone represented by following Formula (4): 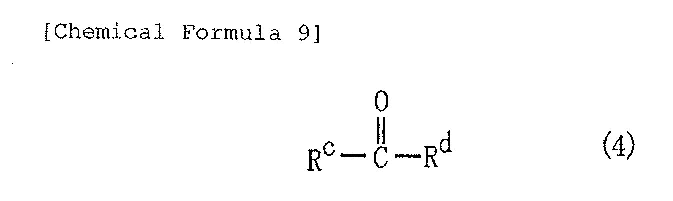 (wherein R^{c} and R^{d} are the same as or different from each other and each represent an organic group having a carbon atom at a bonding site with the adjacent carbon atom, or R^{c} and R^{d} may be combined to form a ring together with the adjacent carbon atom),
the secondary alcohol represented by following Formula (5): 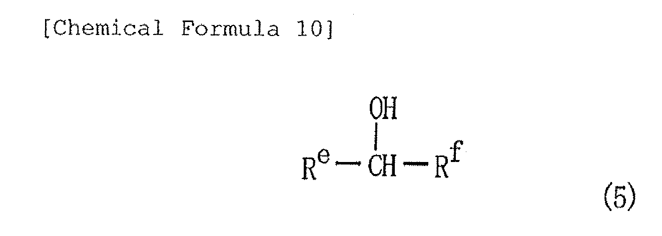 (wherein R^{e} and R^{f} are the same as or different from each other and each represent an organic group having a carbon atom at a bonding site with the adjacent carbon atom, and wherein R^{e} and R^{f} may be combined to form a ring together with the adjacent carbon atom),
the nitrogen-containing cyclic compound containing, as a constituent of its ring, a skeleton represented by following Formula (I): 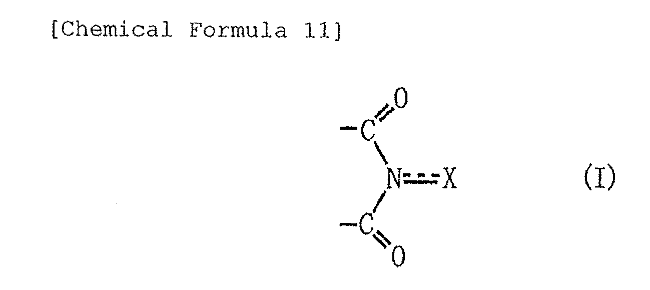 [wherein X represents an oxygen atom or an -OR group where R represents a hydrogen atom or a hydroxyl-protecting group], to give a compound represented by following Formula (6): 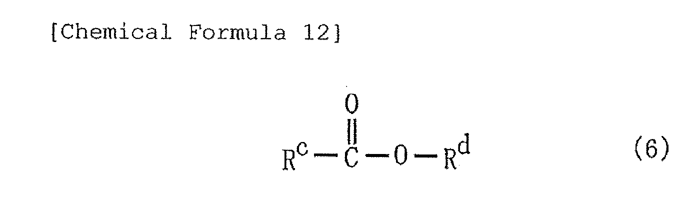 (wherein R^{c} and R^{d} are the same as or different from each other and each represent an organic group having a carbon atom at a bonding site with the adjacent carbonyl-carbon atom or oxygen atom, or R^{c} and R^{d} may be combined to form a ring together with the adjacent carbonyl-carbon atom and oxygen atom). This method is hereinafter also referred to as "fourth production method of esters or lactones".

The present invention further provides a method for producing an ester or lactone, which method includes the steps of: oxidizing a ketone by molecular oxygen in the presence of a secondary alcohol and a nitrogen-containing cyclic compound, the ketone represented by following Formula (4): 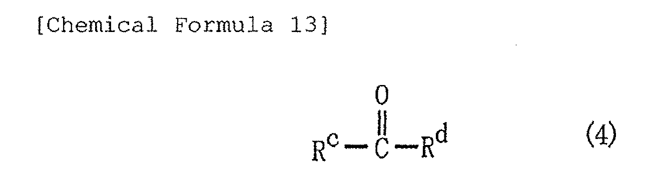 (wherein R^{c} and R^{d} are the same as or different from each other and each represent an organic group having a carbon atom at a bonding site with the adjacent carbon atom, or R^{c} and R^{d} may be combined to form a ring together with the adjacent carbon atom),
the secondary alcohol represented by following Formula (5): 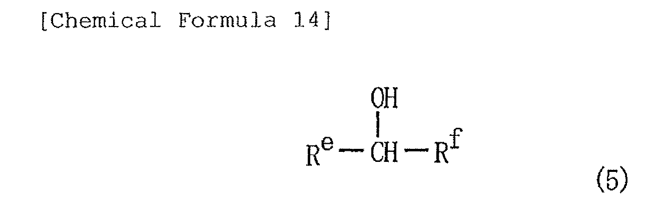 (wherein R^{e} and R^{f} are the same as or different from each other and each represent an organic group having a carbon atom at a bonding site with the adjacent carbon atom, and wherein R^{e} and R^{f} may be combined to form a ring together with the adjacent carbon atom),
the nitrogen-containing cyclic compound containing, as a constituent of its ring, a skeleton represented by following Formula (I): 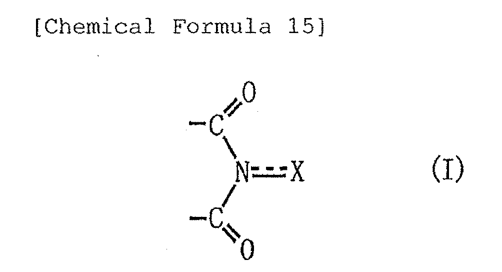 [wherein X represents an oxygen atom or an -OR group where R represents a hydrogen atom or a hydroxyl-protecting group]; and treating the oxidation product with at least one of a fluorine-containing alcohol and a fluorinated sulfonic acid, to give a compound represented by following Formula (6): 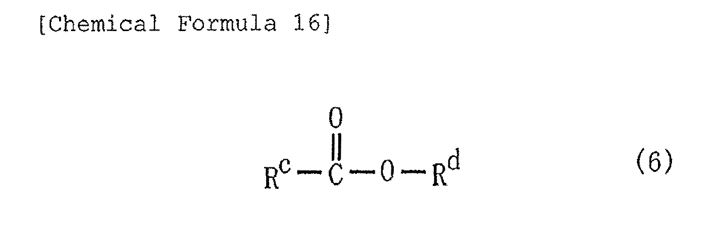 (wherein R^{e} and R^{d} are the same as or different from each other and each represent an organic group having a carbon atom at a bonding site with the adjacent carbonyl-carbon atom or oxygen atom, or R^{e} and R^{d} may be combined to form a ring together with the adjacent carbonyl-carbon atom and oxygen atom). The method is hereinafter also referred to as "fifth production method of esters or lactones".

As used herein a "secondary alcohol" in the first or second production method of esters or lactones, and a "ketone" in the fourth or fifth production method of esters or lactones are each also simply referred to as a "substrate".

### Advantages

The present invention enables industrially efficient production of corresponding esters or lactones from secondary alcohols or oxidation products thereof (dimerized peroxides corresponding to the secondary alcohols) in a high selectivity through a simple and easy operation.

In addition, the present invention enables industrially efficient production of corresponding esters or lactones from ketones in a high selectivity through a simple and easy operation.

### Best Modes for Carrying Out the Invention

In the first production method of esters or lactones according to the present invention, a secondary alcohol represented by Formula (1) is oxidized by molecular oxygen in the presence of a nitrogen-containing cyclic compound containing a skeleton represented by Formula (I) as a constituent of its ring and at least one of a fluorine-containing alcohol and a fluorinated sulfonic acid, to give an ester or lactone represented by Formula (2). In the second production method of esters or lactones according to the present invention, a secondary alcohol represented by Formula (1) is oxidized by molecular oxygen in the presence of a nitrogen-containing cyclic compound containing a skeleton represented by Formula (I) as a constituent of its ring and is then treated with at least one of a fluorine-containing alcohol and a fluorinated sulfonic acid to give an ester or lactone represented by Formula (2). In the third production method of esters or lactones according to the present invention, a peroxide represented by Formula (3a) and/or (3b) is treated with at least one of a fluorine-containing alcohol and a fluorinated sulfonic acid to give an ester or lactone represented by Formula (2). In the fourth production method of esters or lactones according to the present invention, a ketone represented by Formula (4) is oxidized by molecular oxygen in the presence of a secondary alcohol represented by Formula (5), a nitrogen-containing cyclic compound containing a skeleton represented by Formula (I) as a constituent of its ring, and at least one of a fluorine-containing alcohol and a fluorinated sulfonic acid, to give an ester or lactone represented by Formula (6). In the fifth production method of esters or lactones according to the present invention, a ketone represented by Formula (4) is oxidized by molecular oxygen in the presence of a secondary alcohol represented by Formula (5) and a nitrogen-containing cyclic compound containing a skeleton represented by Formula (I) as a constituent of its ring and is then treated with at least one of a fluorinated atom-containing alcohol and a fluorinated sulfonic acid, to give an ester or lactone represented by Formula (6).

### [Secondary Alcohols]

In secondary alcohols represented by Formulae (1) and (5), "organic groups having a carbon atom at a bonding site with the adjacent carbon atom," represented by R^{a}, R^{b}, R^{e}, and R^{f} include hydrocarbon groups and heterocyclic groups. Exemplary hydrocarbon groups include aliphatic hydrocarbon groups (alkyl groups, alkenyl groups, and alkynyl groups) having about 1 to about 20 carbon atoms, such as methyl, ethyl, propyl, isopropyl, butyl, isobutyl, s-butyl, t-butyl, pentyl, neopentyl, hexyl, octyl, decyl, dodecyl, pentadecyl, vinyl, allyl, 1-hexenyl, ethynyl, and 1-butynyl groups, of which those having about 1 to 15 carbon atoms are preferred, and those having about 1 to about 10 carbon atoms are more preferred; alicyclic hydrocarbon groups (cycloalkyl groups and cycloalkenyl groups) having about 3 to about 20 members, such as cyclopropyl, cyclobutyl, cyclopentyl, cyclopentenyl, cyclohexyl, cyclohexenyl, cyclooctyl, and cyclododecyl groups, of which those having about 3 to about 15 members are preferred, and those having about 5 to about 8 members are more preferred; and aromatic hydrocarbon groups having about 6 to about 18 carbon atoms, such as phenyl and naphthyl groups.

Exemplary heterocyclic rings corresponding to the heterocyclic groups include heterocyclic rings containing oxygen atom(s) as heteroatom(s), such as tetrahydrofuran, chroman, isochroman, furan, oxazole, isoxazole, 4-oxo-4H-pyran, benzofuran, isobenzofuran, and 4-oxo-4H-chromene; heterocyclic rings containing sulfur atom(s) as heteroatom(s), such as thiophene, thiazole, isothiazole, thiadiazole, 4-oxo-4H-thiopyran, and benzothiophene; heterocyclic rings containing nitrogen atom(s) as heteroatom(s), such as pyrrolidine, piperidine, piperazine, morpholine, indoline, pyrrole, pyrazole, imidazole, triazole, pyridine, pyridazine, pyrimidine, pyrazine, indole, quinoline, acridine, naphthyridine, quinazoline, and purine.

R^{a} and R^{b}, or R^{e} and R^{f} may be combined to form a ring together with the adjacent carbon atom. Examples of the ring include alicyclic hydrocarbon rings (cycloalkane rings and cycloalkene rings) having about 3 to about 20 members, such as cyclopropane, cyclobutane, cyclopentane, cyclopentene, cyclohexane, cyclohexene, cyclooctane, and cyclododecane rings, of which those having about 3 to about 15 members are preferred, and those having about 3 to about 12 members are more preferred; bridged hydrocarbon rings and bridged heterocyclic rings having about two to four rings, such as norbornane ring, norbornene ring, and adamantane ring; and nonaromatic heterocyclic rings having about 5 to about 8 members, such as tetrahydrofuran, chroman, isochroman, pyrrolidine, and piperidine.

Each of the organic groups and the rings, formed by R^{a} and R^{b} or by R^{e} and R^{f} with the adjacent carbon atom, may have one or more substituents. Exemplary substituents include halogen atoms, hydroxyl group, mercapto group, oxo group, substituted oxy groups (e.g., alkoxy groups, aryloxy groups, and acyloxy groups), substituted thio groups, carboxyl group, substituted oxycarbonyl groups, substituted or unsubstituted carbamoyl groups, cyano group, nitro group, substituted or unsubstituted amino groups, sulfo group, alkyl groups (e.g., alkyl groups having 1 to 4 carbon atoms, such as methyl, ethyl, and t-butyl groups), alkenyl groups (e.g., alkenyl groups having 2 to 4 carbon atoms), alkynyl groups (e.g., alkynyl groups having 2 to 4 carbon atoms), alicyclic hydrocarbon groups, aromatic hydrocarbon groups, and heterocyclic groups. The rings may each have an aromatic or nonaromatic ring (hydrocarbon ring or heterocyclic ring) fused thereto.

Representative examples of secondary alcohols represented by Formulae (1) and (5) include aliphatic secondary alcohols such as 2-propanol, 2-butanol, 3-methyl-2-butanol, 4-methyl-2-pentanol, 3-methyl-2-pentanol, 3,3-dimethyl-2-butanol, 2-dodecanol, 2-methyl-3-pentanol, 2-methyl-3-heptanol, 1-buten-3-ol, 2-methyl-l-buten-3-ol, 1-cyclohexylethanol, 1-phenylethanol, 1-(2-methylphenyl)ethanol, 1-(2-pyridyl)ethanol, 1-cyclohexyl-1-phenylmethanol, benzhydrol (diphenylmethanol), and α-phenethyl alcohol; and alicyclic alcohols such as cyclopropanol, cyclobutanol, cyclopentanol, cyclohexanol, 4-methylcyclohexanol, 4-chlorocyclohexanol, 2,4,4-trimethylcyclohexen-6-ol, cycloheptanol, cyclooctanol, cyclodecanol, cyclododecanol, cyclopentadecanol, 1,3-cyclohexanediol, 1,4-cyclohexanediol, 1,4-cyclooctanediol, 2,2-bis(4-hydroxycyclohexyl)propane, bis(4-hydroxyhexyl)methane, 4-(4-hydroxycyclohexyl)cyclohexanol, and 2-adamantanol.

The amount of the secondary alcohol represented by Formula (5) in the fourth or fifth production method of esters or lactones is, for example, from about 0.1 to about 10 moles, preferably from about 0.1 to about 7 moles, and more preferably from about 1.1 to about 5 moles, per 1 mole of the substrate.

### [Nitrogen-Containing Cyclic Compounds]

Nitrogen-containing cyclic compounds containing a skeleton represented by Formula (I) as a constituent of their ring are used as catalysts in the production methods according to the present invention. In Formula (I), the bond between the nitrogen atom and X is a single bond or double bond. X represents an oxygen atom or an -OR group, in which R is a hydrogen atom or a hydroxyl-protecting group. The nitrogen-containing cyclic compound may have two or more skeletons represented by Formula (I) per molecule. When X is an -OR group and R is a hydroxyl-protecting group in the nitrogen-containing cyclic compounds, two or more of the moiety of the skeleton represented by Formula (I), in which X is an -OR group, other than R may be combined with each other through R.

The hydroxyl-protecting group represented by R in Formula (I) can be any of hydroxyl-protecting groups commonly used in organic synthesis. Examples of such protecting groups include alkyl groups (e.g., alkyl groups having 1 to 4 carbon atoms, such as methyl and t-butyl groups), alkenyl groups (e.g., allyl group), cycloalkyl groups (e.g., cyclohexyl group), aryl groups (e.g., 2,4-dinitrophenyl group), aralkyl groups (e.g., benzyl, 2,6-dichlorobenzyl, 3-bromobenzyl, 2-nitrobenzyl, and triphenylmethyl groups); groups capable of forming an acetal or hemiacetal group with hydroxyl group, including substituted methyl groups (e.g., methoxymethyl, methylthiomethyl, benzyloxymethyl, t-butoxymethyl, 2-methoxyethoxymethyl, 2,2,2-trichloraethoxymethyl, bins(2-chloroethoxy)methyl, and 2-(trimethylsilyl)ethoxymethyl groups), substituted ethyl groups (e.g., 1-ethoxyethyl, 1-methyl-1-methoxyethyl, 1-isopropoxyethyl, 2,2,2-trichloroethyl, and 2-methoxyethyl groups), tetrahydropyranyl group, tetrahydrofuranyl group, and 1-hydroxyalkyl groups (e.g., 1-hydroxyethyl, 1-hydroxyhexyl, 1-hydroxydecyl, 1-hydroxyhexadecyl, and 1-hydroxy-1-phenylmethyl groups); acyl groups (e.g., aliphatic saturated or unsaturated acyl groups including aliphatic acyl groups having 1 to 20 carbon atoms, such as formyl, acetyl, propionyl, butyryl, isobutyryl, valeryl, pivaloyl, hexanoyl, heptanoyl, octanoyl, nonanoyl, decanoyl, lauroyl, myristoyl, palmitoyl, and stearoyl groups; acetoacetyl group; alicyclic acyl groups including cycloalkanecarbonyl groups such as cyclopentanecarbonyl and cyclohexanecarbonyl groups; and aromatic acyl groups such as benzoyl and naphthoyl groups), sulfonyl groups (e.g., methanesulfonyl, ethanesulfonyl, trifluoromethanesulfonyl, benzenesulfonyl, p-toluenesulfonyl, and naphthalenesulfonyl groups), alkoxycarbonyl groups (e.g., alkoxy-carbonyl groups whose alkoxy moiety having 1 to 4 carbon atoms, such as methoxycarbonyl, ethoxycarbonyl, and t-butoxycarbonyl groups), aralkyloxycarbonyl groups (e.g., benzyloxycarbonyl group and p-methoxybenzyloxycarbonyl group), substituted or unsubstituted carbamoyl groups (e.g., carbamoyl, methylcarbamoyl, and phenylcarbamoyl groups), groups corresponding to inorganic acids (e.g., sulfuric acid, nitric acid, phosphoric acid, and boric acid), except for removing hydroxyl group (OH group) therefrom, dialkylphosphinothioyl groups (e.g., dimethylphosphinothioyl group), diarylphosphinothioyl groups (e.g., diphenylphosphinothioyl group), and substituted silyl groups (e.g., trimethylsilyl, t-butyldimethylsilyl, tribenzylsilyl, and triphenylsilyl groups).

When X is an -OR group, and two or more of the moiety of the skeleton represented by Formula (I) other than R are combined through R, examples of the Rs include acyl groups of polycarboxylic acids, such as oxalyl, malonyl, succinyl, glutaryl, phthaloyl, isophthaloyl, and terephthaloyl groups; carbonyl group; and multivalent hydrocarbon groups such as methylene, ethylidene, isopropylidene, cyclopentylidene, cyclohexylidene, and benzylidene groups, of which groups capable of forming acetal bonds with two hydroxyl groups are preferred.

Preferred examples of R include hydrogen atom; groups capable of forming acetal or hemiacetal group(s) with hydroxyl group(s); groups corresponding to acids such as carboxylic acid, sulfonic acid, carbonic acid, carbamic acid, sulfuric acid, phosphoric acid, and boric acid, except for removing hydroxyl group (OH group) therefrom (e.g., acyl groups, sulfonyl groups, alkoxycarbonyl groups, and carbamoyl groups), and other hydrolyzable protecting groups capable of removing through hydrolysis. Among them, hydrogen atom is especially preferred as R.

The nitrogen-containing cyclic compounds containing a skeleton represented by Formula (I) as a constituent of their ring include:
cyclic imide compounds represented by following Formula (7): 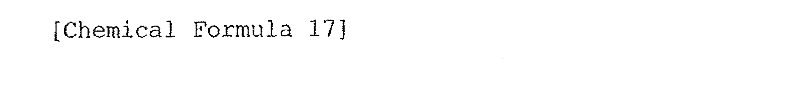 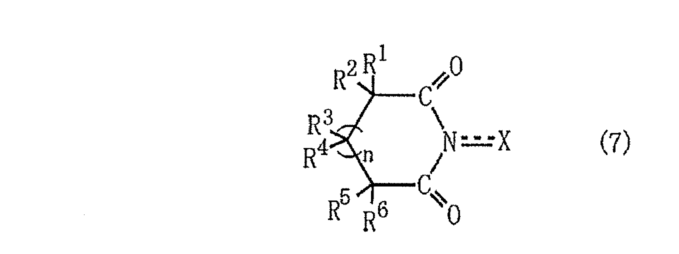 [wherein "n" denotes 0 or 1; X represents an oxygen atom or an -OR group, wherein R represents a hydrogen atom or a hydroxyl-protecting group; R¹, R², R³, R⁴, R⁵, and R⁶ are the same as or different from one another and each represent a hydrogen atom, a halogen atom, an alkyl group, an aryl group, a cycloalkyl group, a hydroxyl group, an alkoxy group, a carboxyl group, a substituted oxycarbonyl group, an acyl group, or an acyloxy group, or at least two of R¹, R², R³, R⁴, R⁵, and R⁶ may be combined to form a double bond or an aromatic or nonaromatic ring together with a carbon atom or carbon-carbon bond constituting the cyclic imide skeleton, wherein one or more of an N-substituted cyclic imide group represented by following Formula (a): 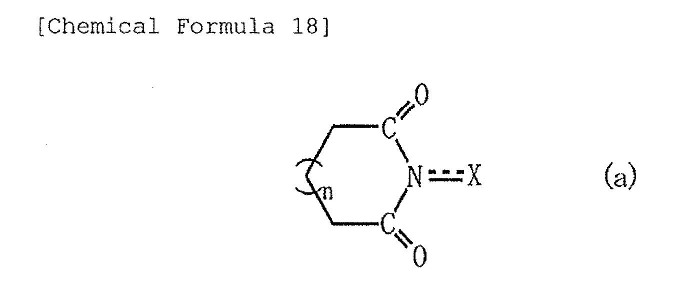 (wherein "n" and X are as defined above)
   may further be formed on the substituents R¹, R², R³, R⁴, R⁵, and R⁶, or on the double bond or aromatic or nonaromatic ring formed by at least two of R¹, R², R³, R⁴, R⁵, and R⁶]

In the cyclic imide compounds represented by Formula (7), exemplary halogen atoms as the substituents R¹, R², R³, R⁴, R⁵, and R⁶ include iodine, bromine, chlorine, and fluorine atoms. Exemplary alkyl groups include straight- or branched-chain alkyl groups having about 1 to about 30 carbon atoms, such as methyl, ethyl, propyl, isopropyl, butyl, hexyl, decyl, and dodecyl groups, of which those having about 1 to about 20 carbon atoms are preferred.

Exemplary aryl groups include phenyl, tolyl, xylyl, and naphthyl groups; and exemplary cycloalkyl groups include cyclopentyl and cyclohexyl groups. Exemplary alkoxy groups include alkoxy groups having about 1 to about 30 carbon atoms, such as methoxy, ethoxy, isopropoxy, butoxy, t-butoxy, hexyloxy, decyloxy, and dodecyloxy groups, of which those having about 1 to about 20 carbon atoms are preferred.

Exemplary substituted oxycarbonyl groups include alkoxy-carbonyl groups whose alkoxy moiety having 1 to 30 carbon atoms, such as methoxycarbonyl, ethoxycarbonyl, isopropoxycarbonyl, butoxycarbonyl, t-butoxycarbonyl, hexyloxycarbonyl, and decyloxycarbonyl groups, of which alkoxy-carbonyl groups whose alkoxy moiety having 1 to 20 carbon atoms are preferred; cycloalkyloxycarbonyl groups such as cyclopentyloxycarbonyl and cyclohexyloxycarbonyl groups, of which cycloalkyloxycarbonyl groups having 3 to 20 members are preferred; aryloxycarbonyl groups such as phenyloxycarbonyl group, of which aryloxy-carbonyl groups whose aryloxy moiety having 6 to 20 carbon atoms are preferred; and aralkyloxycarbonyl groups such as benzyloxycarbonyl group, of which aralkyloxy-carbonyl groups whose arakyloxy moiety having 7 to 21 carbon atoms are preferred.

Exemplary acyl groups include aliphatic saturated or unsaturated acyl groups including aliphatic acyl groups having 1 to 30 carbon atoms, such as formyl, acetyl, propionyl, butyryl, isobutyryl, valeryl, pivaloyl, hexanoyl, decanoyl, and lauroyl groups, of which aliphatic acyl groups having 1 to 20 carbon atoms are preferred; acetoacetyl group; alicyclic acyl groups including cycloalkanecarbonyl groups such as cyclopentanecarbonyl and cyclohexanecarbonyl groups; and aromatic acyl groups such as benzoyl group.

Exemplary acyloxy groups include aliphatic saturated or unsaturated acyloxy groups including aliphatic acyloxy groups having 1 to 30 carbon atoms, such as formyloxy, acetyloxy, propionyloxy, butyryloxy, isobutyryloxy, valeryloxy, pivaloyloxy, decanoyloxy, and lauroyloxy groups, of which aliphatic acyloxy groups having 1 to 20 carbon atoms are preferred; acetoacetyloxy group; alicyclic acyloxy groups including cycloalkanecarbonyloxy groups such as cyclopentanecarbonyloxy and cyclohexanecarbonyloxy groups; and aromatic acyloxy groups such as benzoyloxy group.

The substituents R¹, R², R³, R⁴, R⁵, and R⁶ may be the same as or different from one another. At least two of R¹, R², R³, R⁴, R⁵, and R⁶ in Formula (7) may be combined to form a double bond or an aromatic or nonaromatic ring together with a carbon atom or carbon-carbon bond constituting the cyclic imide skeleton. Preferred aromatic or nonaromatic rings are rings having about 5 to about 12 members, of which rings having about 6 to about 10 members are more preferred. The ring may be a heterocyclic or fused heterocyclic ring but is often a hydrocarbon ring. Examples of such rings include alicyclic rings (e.g., substituted or unsubstituted cycloalkane rings such as cyclohexane ring; and substituted or unsubstituted cycloalkene rings such as cyclohexene ring), bridged rings (e.g., substituted or unsubstituted bridged hydrocarbon rings such as 5-norbornene ring), and substituted or unsubstituted aromatic rings (including fused rings), such as benzene ring and naphthalene ring. The ring is often composed of an aromatic ring. The ring may have one or more substituents. Exemplary substituents include alkyl groups, haloalkyl groups, hydroxyl group, alkoxy groups, carboxyl group, substituted oxycarbonyl groups, acyl groups, acyloxy groups, nitro group, cyano group, amino group, and halogen atoms.

One or more cyclic imide groups represented by Formula (a) may further be formed on the substituents R¹, R², R³, R⁴, R⁵, and R⁶, or on the double bond or aromatic or nonaromatic ring formed by at least two of R¹, R², R³, R⁴, R⁵, and R⁶. For example, when R¹, R², R³, R⁴, R⁵ or R⁶ is an alkyl group having 2 or more carbon atoms, the cyclic imide group may be formed as including adjacent two carbon atoms constituting the alkyl group. When at least two of R¹, R², R³, R⁴, R⁵, and R⁶ are combined to form a double bond together with a carbon-carbon bond constituting the cyclic imide skeleton, the cyclic imide group may be formed as including the double bond. When at least two of R¹, R², R³, R⁴, R⁵, and R⁶ are combined to form an aromatic or nonaromatic ring together with a carbon atom or carbon-carbon bond constituting the cyclic imide skeleton, the cyclic imide group may be formed as including adjacent two carbon atoms constituting the ring.

Preferred cyclic imide compounds include compounds represented by following formulae: 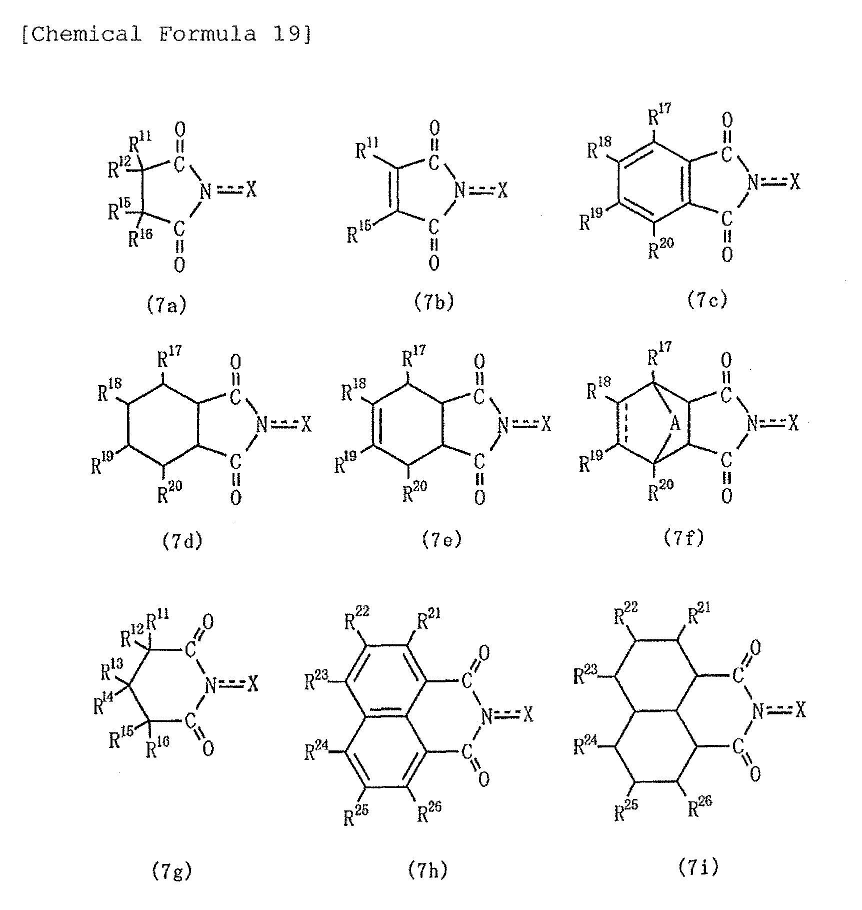 wherein R¹¹, R¹², R¹³, R¹⁴, R¹⁵, and R²⁶ are the same as or different from one another and each represent a hydrogen atom, a halogen atom, an alkyl group, an aryl group, a cycloalkyl group, a hydroxyl group, an alkoxy group, a carboxyl group, a substituted oxycarbonyl group, an acyl group, or an acyloxy group; R¹⁷, R¹⁸, R¹⁹, R²⁰, R²¹, R²², R²³, R²⁴, R²⁵, and R²⁶ are the same as or different from one another and each represent a hydrogen atom, an alkyl group, a haloalkyl group, a hydroxyl group, an alkoxy group, a carboxyl group, a substituted oxycarbonyl group, an acyl group, an acyloxy group, a nitro group, a cyano group, an amino group, or a halogen atom, or adjacent groups among R¹⁷ to R²⁶ may be combined to form a five- or six-membered N-substituted cyclic imide skeleton shown in Formula (7c), (7d), (7e), (7f), (7h), or (7i), and "A" in Formula (7f) represents a methylene group or an oxygen atom; and X is as defined above.

Examples of the halogen atoms, alkyl groups, aryl groups, cycloalkyl groups, hydroxyl group, alkoxy groups, carboxyl group, substituted oxycarbonyl groups, acyl groups, and acyloxy groups as the substituents R¹¹ to R¹⁶ are as the corresponding groups exemplified in the substituents R¹ to R⁶.

In the substituents R¹⁷ to R²⁶, exemplary alkyl groups include alkyl groups as with the above-exemplified alkyl groups, of which alkyl groups having about 1 to about 6 carbon atoms are preferred; exemplary haloalkyl groups include haloalkyl groups having about 1 to about 4 carbon atoms, such as trifluoromethyl group; exemplary alkoxy groups include alkoxy groups as above, of which lower alkoxy groups having about 1 to about 4 carbon atoms are preferred; and exemplary substituted oxycarbonyl groups include substituted oxycarbonyl groups as above, such as alkoxycarbonyl groups, cycloalkyloxycarbonyl groups, aryloxycarbonyl groups, and aralkyloxycarbonyl groups. Exemplary acyl groups include acyl groups as above, including aliphatic saturated or unsaturated acyl groups, acetoacetyl group, alicyclic acyl groups, and aromatic acyl groups; and exemplary acyloxy groups include acyloxy groups as above, including aliphatic saturated or unsaturated acyloxy groups, acetoacetyloxy group, alicyclic acyloxy groups, and aromatic acyloxy groups. Exemplary halogen atoms include fluorine, chlorine, and bromine atoms. Each of the substituents R¹⁷ to R²⁶ is often independently hydrogen atom, a lower alkyl group having about 1 to about 4 carbon atoms, carboxyl group, a substituted oxycarbonyl group, nitro group, or a halogen atom.

Of preferred imide compounds, representative examples of compounds having a five-membered N-substituted cyclic imide skeleton include compounds of Formula (7) in which X is an -OR group and R is a hydrogen atom, such as N-hydroxysuccinimide, N-hydroxy-α-methylsuccinimide, N-hydroxy-α,α-dimethylsuccinimide, N-hydroxy-α,β-dimethylsuccinimide, N-hydroxy-α,α,β,β-tetramethylsuccinimide, N-hydroxymaleimide, N-hydroxyhexahydrophthalimide, N,N'-dihydroxycyclohexanetetracarboxylic diimide, N-hydroxyphthalimide, N-hydroxytetrabromophthalimide, N-hydroxytetrachlorophthalimide, N-hydroxy-HET acid imide (N-hydraxy-2,4,5,6,7,7-hexachloro-5-norbornene-2,3-dicarboximide), N-hydroxy-HIMIC acid imide (N-hydroxy-5-norbornene-2,3-dicarboximide), N-hydroxytrimellitimide, N,N'-dihydroxypyromellitic diimide, N,N'-dihydroxynaphthalenetetracarboxylic diimide, α,β-diacetoxy-N-hydroxysuccinimide, N-hydroxy-α,β-bis(propionyloxy)succinimide, N-hydroxy-α,β-bis(valeryloxy)succinimide, N-hydroxy-α,β-bis(lauroyloxy)succinimide, α,β-bis(benzoyloxy)-N-hydroxysuccinimide, N-hydroxy-4-methoxycarbonylphthalimide, 4-chloro-N-hydroxyphthalimide, 4-ethoxycarbonyl-N-hydroxyphthalimide, N-hydroxy-4-pentyloxycarbonylphthalimide, 4-dodecyloxy-N-hydroxycarbonylphthalimide, N-hydroxy-4-phenoxycarbonylphthalimide, N-hydroxy-4,5-bis(methoxycarbonyl)phthalimide, 4,5-bis(ethoxycarbonyl)-N-hydroxyphthalimide, N-hydroxy-4,5-bis(pentyloxycarbonyl)phthalimide, 4,5-bis(dodecyloxycarbonyl)-N-hydroxyphthalimide, and N-hydroxy-4,5-bis(phenoxycarbonyl)phthalimide; compounds corresponding to these compounds, except that R is an acyl group such as acetyl group, propionyl group, or benzoyl group; compounds of Formula (7) in which X is an -OR group and R is a group capable of forming acetal or hemiacetal bond(s) with hydroxyl group(s), such as N-methoxymethyloxyphthalimide, N-(2-methoxyethoxymethyloxy)phthalimide, and N-tetrahydropyranyloxyphthalimide; compounds of Formula (7) in which X is an -OR group and R is a sulfonyl group, such as N-methanesulfonyloxyphthalimide and N-(p-toluenesulfonyloxy)phthalimide; and compounds of Formula (7) in which X is an -OR group and R is a group corresponding to an inorganic acid, except for removing hydroxyl group (OH group) therefrom, such as sulfuric acid ester, nitric acid ester, phosphoric ester, or boric acid ester of N-hydroxyphthalimide.

Of preferred imide compounds, representative examples of compounds having a six-membered N-substituted cyclic imide skeleton include compounds of Formula (7) in which X is an -OR group and R is a hydrogen atom, such as N-hydroxyglutarimide, N-hydroxy-α,α-dimethylglutarimide, N-hydroxy-β,β-dimethylglutarimide, N-hydroxy-1,8-decahydronaphthalenedicarboximide, N,N'-dihydroxy-1,8;4,5-decahydronaphthalenetetracarboxylic diimide, N-hydroxy-1,8-naphthalenedicarboximide (N-hydroxynaphthalimide), and N,N'-dihydroxy-1,8;4,5-naphthalenetetracarboxylic diimide; compounds corresponding to these compounds, except that R is an acyl group such as acetyl group, propionyl group, or benzoyl group; compounds of Formula (7) in which X is an -OR group and R is a group capable of forming acetal or hemiacetal bond(s) with hydroxyl group(s), such as N-methoxymethyloxy-1,8-naphthalenedicarboximide and N,N'-bis(methoxymethyloxy)-1,8;4,5-naphthalenetetracarboxylic diimide; compounds of Formula (7) in which X is an -OR group and R is a sulfonyl group, such as N-methanesulfonyloxy-1,8-naphthalenedicarboximide and N,N'-bis(methanesulfonyloxy)-1,8;4,5-naphthalenetetracarboxylic diimide; and compounds of Formula (7) in which X is an -OR group and R is a group corresponding to an inorganic acid, except for removing OH group therefrom, such as a sulfuric acid ester, nitric acid ester, phosphoric ester, or boric acid ester of N-hydroxy-1,8-naphthalenedicarboximide or N,N'-dihydroxy-1,8;9,5-naphthalenetetracarboxylic diimide.

The nitrogen-containing cyclic compounds containing a skeleton represented by Formula (I) as a constituent of their ring further include, in addition to the cyclic imide compounds, cyclic acylurea compounds having a cyclic acylurea skeleton [-C(=O)-N-C(=O)-N-]. Representative examples of such cyclic acylurea compounds include hydro-1-hydroxy (or 1-substituted-oxy)-1,3,5-triazine-2,6-dione compounds represented by following Formula (8): 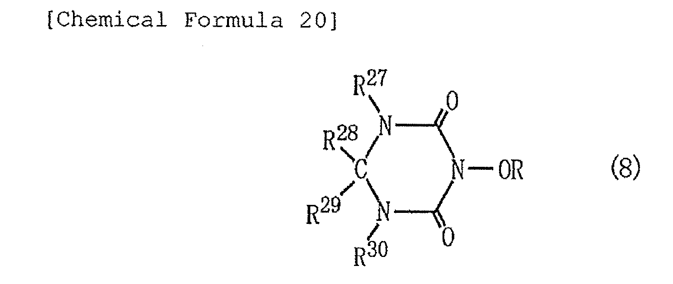 wherein R²⁷ and R³⁰ are the same as or different from each other and each represent a hydrogen atom, an alkyl group, an aryl group, a cycloalkyl group, a protected or unprotected hydroxyl group, a protected or unprotected carboxyl group, or an acyl group; R²⁸ and R²⁹ are the same as or different from each other and each represent a hydrogen atom, a halogen atom, an alkyl group, an aryl group, a cycloalkyl group, a hydroxyl group, an alkoxy group, a carboxyl group, a substituted oxycarbonyl group, an acyl group, or an acyloxy group, or at least two of R²⁷, R²⁸, R²⁹, and R³⁰ may be combined to form a double bond or an aromatic or nonaromatic ring together with an atom constituting the ring in Formula (8), or R²⁸ and R²⁹ may together form an oxo group; and R is as defined above.

In Formula (8), examples of alkyl groups, aryl groups, cycloalkyl groups, and acyl groups as R²⁷ and R³⁰ are as with the alkyl groups and other groups exemplified in the substituents R¹ to R⁶. Exemplary hydroxyl-protecting groups are as above.

Exemplary carboxyl-protecting groups include protecting groups commonly used in organic synthesis, including alkoxy groups (e.g., alkoxy groups having 1 to 6 carbon atoms, such as methoxy, ethoxy, and butoxy), cycloalkyloxy groups, aryloxy groups (e.g., phenoxy group), aralkyloxy groups (e.g., benzyloxy group), trialkylsilyloxy groups (e.g., trimethylsilyloxy group), substituted or unsubstituted amino groups (e.g., amino group; mono- or di-(alkyl)amino groups whose alkyl moiety having 1 to 6 carbon atoms, such as methylamino group and dimethylamino group).

Examples of halogen atoms, alkyl groups, aryl groups, cycloalkyl groups, hydroxyl group, alkoxy groups, carboxyl group, substituted oxycarbonyl groups, acyl groups, and acyloxy groups as R²⁸ and R²⁹ are as with the alkyl groups and other groups exemplified in the substituents R¹ to R⁶.

In Formula (8), at least two of R²⁷, R²⁸, R²⁹, and R³⁰ may be combined to form a double bond or an aromatic or nonaromatic ring together with an atom (carbon atom and/or nitrogen atom) constituting the ring in Formula (8); and R²⁸ and R²⁹ may together form an oxo group. Preferred examples of the aromatic or nonaromatic ring are as above.

Of the compounds represented by Formula (8), preferred are isocyanuric acid derivatives represented by following Formula (8a): 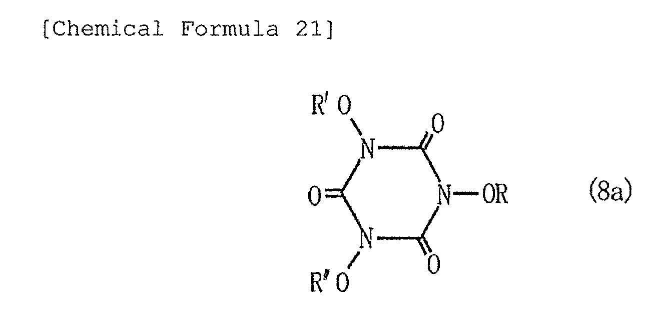 wherein R, R', and R" are the same as or different from one another and each represent a hydrogen atom or a hydroxyl-protecting group.

Examples of representative compounds belonging to the cyclic acylurea compounds include hexahydro-1,3,5-trihydroxy-1,3,5-triazine-2,4,6-trione (i.e., 1,3,5-trihydroxyisocyanuric acid), 1,3,5-triacetoxy-hexahydro-1,3,5-triazine-2,4,6-trione, hexahydro-1,3,5-tris(methoxymethyloxy)-1,3,5-triazine-2,4,6-trione, hexahydxa-1-hydroxy-1,3,5-triazine-2,6-dione, hexahydro-1-hydroxy-3,5-dimethyl-1,3,5-triazine-2,6-dione, I-acetoxy-hexahydro-1,3,5-triazine-2,6-dione, and 1-acetoxy-hexahydro-3,5-dimethyl-1,3,5-triazine-2,6-dione.

Among the nitrogen-containing cyclic compounds, compounds in which X is an -OR group and R is a hydrogen atom (N-hydroxy cyclic compounds) can be prepared according to a known process or a combination of known processes. Among the nitrogen-containing cyclic compounds, compounds in which X is an -OR group and R is a hydroxyl-protecting group can be prepared by introducing a desired protecting group into corresponding compounds in which R is a hydrogen atom (N-hydroxy cyclic compounds) using a common reaction for introducing such protecting groups.

More specifically, among the cyclic imide compounds, compounds in which X is an -OR group and R is a hydrogen atom (N-hydroxy cyclic imide compounds) can be prepared by a common imidization process (a process for the formation of an imide), such as a process that includes the steps of reacting a corresponding acid anhydride with hydroxylamine for ring-opening of the acid anhydride group, and closing the ring to form an imide. N-acetoxyphthalimide, for example, can be prepared by reacting N-hydroxyphthalimide with acetic anhydride or by reacting N-hydroxyphthalimide with an acetyl halide in the presence of a base. The compounds can also be prepared by other processes.

Among them, examples of cyclic imide compounds typically preferred as catalysts include N-hydroxy imide compounds (e.g., N-hydroxysuccinimide, N-hydroxyphthalimide, N,N'-dihydroxypyromellitic diimide, N-hydroxyglutarimide, N-hydroxy-1,8-naphthalenedicarbaximide, and N,N'-dihydroxy-1,8:4,5-naphthalenetetracarbaxylic diimide) derived from aliphatic polycarboxylic acid anhydrides (cyclic anhydrides) or derived from aromatic polycarboxylic acid anhydrides (cyclic anhydrides); and compounds corresponding to these N-hydroxy imide compounds, except for introducing a protecting group into a hydroxyl group thereof.

Among the cyclic acylurea compounds, 1,3,5-triacetoxy-hexahydro-1,3,5-triazine-2,4,6-trione (i.e., 1,3,5-triacetoxyisocyanuric acid), for example, can be prepared by reacting hexahydro-1,3,5-trihydroxy-1,3,5-triazine-2,4,6-trione (i.e., 1,3,5-trihydroxyisocyanuric acid) with acetic anhydride or by reacting hexahydro-1,3,5-trihydroxy-1,3,5-triazine-2,4,6-trione with an acetyl halide in the presence of a base.

Each of different nitrogen-containing cyclic compounds containing a skeleton represented by Formula (I) as a constituent of their ring can be used alone or in combination. The nitrogen-containing cyclic compounds may be formed within the reaction system. The nitrogen-containing cyclic compounds may be used as being supported by a support (carrier). The support used herein is often a porous support such as activated carbon, zeolite, silica, silica-alumina, or bentonite. The amount of the nitrogen-containing cyclic compound(s) on the support is, for example, from about 0.1 to about 50 parts by weight, preferably from about 0.5 to about 30 parts by weight, and more preferably from about 1 to about 20 parts by weight, per 100 parts by weight of the support.

The amount of the nitrogen-containing cyclic compounds can be chosen within a broad range and is, for example, from about 0.0000001 to about 1 mole, preferably from about 0.0001 to about 0.5 mole, more preferably from about 0.001 to about 0.4 mole, and especially preferably from about 0.01 to about 0.35 mole, per 1 mole of the secondary alcohol represented by Formula (1) or per 1 mole of the ketone represented by Formula (4).

### [Oxygen]

The molecular oxygen for use in the oxidation of the substrate is not specifically limited and can be any of pure oxygen; oxygen diluted with an inert gas such as nitrogen, helium, argon, or carbon dioxide gas; and air.

The amount of the molecular oxygen can be suitably chosen according to the type of the substrate but is, for example, about 0.5 mole or more (e.g., about 1 mole or more), preferably from about 1 to about 100 moles, and more preferably from about 2 to about 50 moles, per 1 mole of the substrate. The molecular oxygen is often used in excess moles to the substrate.

### [Ketone in First or Second Production Method of Esters or Lactones]

The secondary alcohol represented by Formula (1) is preferably oxidized in the presence of a ketone in the first or second production method of esters or lactones according to the present invention. The presence of a ketone in the system enhances the formation of a stable peroxide represented by Formula (3a) and/or (3b) and enables the production of a target compound in a high selectivity through oxidation in the presence of at least one of a fluorine-containing alcohol and a fluorinated sulfonic acid or through a treatment with at least one of a fluorine-containing alcohol and a fluorinated sulfonic acid after oxidation. In Formulae (3a) and (3b), R^{a}s and R^{b}s are as defined above, and two R^{a}s and two R^{b}s in the molecule may differ from each other respectively. The compound represented by Formula (3b) (hydroxyhydroperoxide compound) is probably formed by a reaction of hydrogen peroxide with a ketone (especially a ketone corresponding to the secondary alcohol), which hydrogen peroxide is formed in situ through oxidation of the secondary alcohol by oxygen. The compound represented by Formula (3a) and/or (3b) is easily rearranged into a corresponding ester or lactone by the action of at least one of a fluorine-containing alcohol and a fluorinated sulfonic acid. The third production method of esters or lactones according to the present invention mentioned in detail later is a method for producing a corresponding ester or lactone by treating the peroxide represented by Formula (3a) and/or (3b) with at least one of a fluorine-containing alcohol and a fluorinated sulfonic acid.

The ketone may be added to the system in a single unit or in plural installments. The ketone may be a ketone corresponding to the secondary alcohol represented by Formula (1). The corresponding ketone is represented by following Formula (9):

R^{a}-C(=O)-R^{b} (9)

wherein R^{a} and R^{b} are as defined above.

Specific examples of such ketones include ketones corresponding to the representative examples of the secondary alcohol, including open-chain ketones such as acetone, methyl ethyl ketone, methyl isopropyl ketone, methyl isobutyl ketone, methyl s-butyl ketone, methyl t-butyl ketone, methyl decyl ketone, ethyl isopropyl ketone, isopropyl butyl ketone, methyl vinyl ketone, methyl isopropenyl ketone, methyl cyclohexyl ketone, methyl phenyl ketone, methyl (2-methylphenyl) ketone, methyl (2-pyridyl) ketone, cyclohexyl phenyl ketone, and diphenyl ketone; and cyclic ketones such as cyclopropanone, cyclobutanone, cyclopentanone, cyclohexanone, 4-methylcyclohexanone, 4-chlorocyclohexanone, isophorone, cycloheptanone, cyclooctanone, cyclodecanone, cyclododecanone, cyclopentadecanone, 1,3-cyclohexanedione, 1,4-cyclohexanedione, 1,4-cyclooctanedione, 2,2-bis(4-oxocyclohexyl)propane, bis(4-oxocyclohexyl)methane, 4-(4-oxocyclohexyl)cyclohexanone, and 2-adamantanone.

According to a preferred embodiment of the present invention, a ketone corresponding to the substrate secondary alcohol represented by Formula (1) is used as the ketone. Typically, when cyclohexanol is used as the substrate, cyclohexanone is used as the ketone. In this case, a so-called K/A oil (a mixture of cyclohexanol and cyclohexanone) is advantageously used. The K/A oil can be prepared inexpensively through autoxidation of cyclohexane.

When a secondary alcohol as the substrate is used in combination with a ketone corresponding to the substrate, for example, a peroxide represented by Formula (3a) and/or (3b) has a symmetrical structure in the oxidation reaction (i.e., two R^{a}s are identical groups, and two R^{b}s are identical groups in the molecule); and this can give a single ester or lactone through oxidation in the presence of a fluorine-containing alcohol or through treatment with a fluorine-containing alcohol performed after oxidation. This enables production of a target compound in a good selectivity and easy purification thereof.

Each of different ketones can be used alone or in combination. The amount of ketones is from about 0 to about 10 moles (e.g., from about 0.1 to about 10 moles), preferably from about 0.1 to about 5 moles, and more preferably from about 0.2 to about 0.9 mole, per 1 mole of the substrate.

### [Ketone in Fourth or Fifth Production Method of Esters or Lactones]

A ketone represented by Formula (4) is used as the substrate in the fourth or fifth production method of esters or lactones according to the present invention.

In Formula (4), R^{c} and R^{d} are the same as or different from each other and each represent an organic group having a carbon atom at a bonding site with the adjacent carbon atom, in which R^{c} and R^{d} may be combined to form a ring together with the adjacent carbon atom.

In the ketone represented by Formula (4), the "organic groups having a carbon atom at a bonding site with the adjacent carbon atom" as R^{c} and R^{d} include hydrocarbon groups and heterocyclic groups. Exemplary hydrocarbon groups include aliphatic hydrocarbon groups (alkyl groups, alkenyl groups, and alkynyl groups) having about 1 to about 20 carbon atoms, such as methyl, ethyl, propyl, isopropyl, butyl, isobutyl, s-butyl, t-butyl, pentyl, neopentyl, hexyl, octyl, decyl, dodecyl, pentadecyl, vinyl, allyl, 1-hexenyl, ethynyl, and 1-butynyl groups, of which those having about 1 to about 15 carbon atoms are preferred, and those having about 1 to about 10 carbon atoms are more preferred; alicyclic hydrocarbon groups (cycloalkyl groups and cycloalkenyl groups) having about 3 to about 20 members, such as cyclopropyl, cyclobutyl, cyclopentyl, cyclopentenyl, cyclohexyl, cyclohexenyl, cyclooctyl, and cyclododecyl groups, of which those having about 3 to about 15 members are preferred, and those having about 5 to about 8 members are more preferred; and aromatic hydrocarbon groups having about 6 to about 18 carbon atoms, such as phenyl and naphthyl groups.

Exemplary heterocyclic rings corresponding to the heterocyclic groups include heterocyclic rings containing oxygen atom(s) as heteroatom(s), such as tetrahydrofuran, chroman, isochroman, furan, oxazole, isoxazole, 4-oxo-4H-pyran, benzofuran, isobenzofuran, and 4-oxo-4H-chromene; heterocyclic rings containing sulfur atom(s) as heteroatom(s), such as thiophene, thiazole, isothiazole, thiadiazole, 4-oxo-4H-thiopyran, and benzothiophene; and heterocyclic rings containing nitrogen atom(s) as heteroatom(s), such as pyrrolidine, piperidine, piperazine, morpholine, indoline, pyrrole, pyrazole, imidazole, triazole, pyridine, pyridazine, pyrimidine, pyrazine, indole, quinoline, acridine, naphthyridine, quinazoline, and purine.

Examples of rings which may be formed by R^{c} and R^{d} with the adjacent carbon atom include alicyclic hydrocarbon rings (cycloalkane rings and cycloalkene rings) having about 3 to about 20 members, such as cyclopropane, cyclobutane, cyclopentane, cyclopentene, cyclohexane, cyclohexene, cyclooctane, and cyclododecane rings, of which those having about 3 to about 15 members are preferred, and those having about 3 to about 12 members are more preferred; bridged hydrocarbon rings or bridged heterocyclic rings having about two to about four rings, such as norbornane ring, norbornene ring, and adamantane ring; and nonaromatic heterocyclic rings having about 5 to about 8 members, such as tetrahydrofuran, chroman, isochroman, pyrrolidine, and piperidine.

The organic group, and the ring which may be formed by R^{c} and R^{d} with the adjacent carbon atom may each be substituted. Examples of such substituents include halogen atoms, hydroxyl group, mercapto group, oxo group, substituted oxy groups (e.g., alkoxy groups, aryloxy groups, and acyloxy groups), substituted thio groups, carboxyl group, substituted oxycarbonyl groups, substituted or unsubstituted carbamoyl groups, cyano group, nitro group, substituted or unsubstituted amino groups, sulfo groups, alkyl groups (e.g., alkyl groups having 1 to 4 carbon atoms, such as methyl, ethyl, and t-butyl groups), alkenyl groups (e.g., alkenyl groups having 2 to 4 carbon atoms), alkynyl groups (e.g., alkynyl groups having 2 to 4 carbon atoms), alicyclic hydrocarbon groups, aromatic hydrocarbon groups, and heterocyclic groups. The ring may have an aromatic or nonaromatic ring (hydrocarbon ring or heterocyclic ring) fused thereto.

Examples of ketones represented by Formula (4) include open-chain ketones such as acetone, methyl ethyl ketone, methyl isopropyl ketone, methyl isobutyl ketone, methyl s-butyl ketone, methyl t-butyl ketone, methyl decyl ketone, ethyl isopropyl ketone, isopropyl butyl ketone, methyl vinyl ketone, methyl isopropenyl ketone, methyl cyclohexyl ketone, methyl phenyl ketone, methyl (2-methylphenyl) ketone, methyl (2-pyridyl) ketone, cyclohexyl phenyl ketone, and diphenyl ketone; and cyclic ketones such as cyclopropanone, cyclobutanone, cyclopentanone, cyclohexanone, 4-methylcyclohexanone, 4-chlorocyclohexanone, isophorone, cycloheptanone, cyclooctanone, cyclodecanone, cyclododecanone, cyclopentadecanone, 1,3-cyclohexanedione, 1,4-cyclohexanedione, 1,4-cyclooctanedione, 2,2-bis(4-oxocyclohexyl)propane, bis(4-oxocyclohexyl)methane, 4-(4-oxocyclohexyl)cyclohexanone, and 2-adamantanone. Among them, cyclic ketones are preferably used.

### [Radical Generators]

The secondary alcohol represented by Formula (1) is preferably oxidized in the presence of a radical generator in the methods according to the present invention. The presence of a radical generator in the system helps a target compound to be produced in a high selectivity through oxidation in the presence of at least one of a fluorine-containing alcohol and a fluorinated sulfonic acid or through treatment with at least one of a fluorine-containing alcohol and a fluorinated sulfonic acid performed after oxidation. This is probably because a peroxide represented by Formula (3a) and/or (3b) is selectively formed in the oxidation reaction. The radial generator may be added to the system as a single unit or sequentially. The sequential addition may be performed in two ways, namely, intermittent addition in plural installments and continuous addition in small portions.

Exemplary radical generators include compounds used as initiators for radical polymerization. Representative examples of radical initiators include acetophenones such as 2,2-diethoxyacetophenone; peroxides including diacyl peroxides such as benzoyl peroxide (BPO), ketone peroxides such as cyclohexanone peroxide, peroxyketals such as 1,1-bis(t-butylperoxy)-3,3,5-trimethylcyclohexane, hydroperoxides such as t-hexyl peroxide, peroxy esters such as 1,1,3,3-tetramethylbutyl peroxy-2-ethylhexanoate, and peroxydicarbonates such as diisopropyl peroxydicarbonate; azo compounds such as azobisisobutyronitrile (AIBN), dimethyl-2,2'-azobis(isobutyrate) (MAIB), and dibutyl-2,2'-azobisisobutylate; and cyclic amine-N-oxyl compounds such as 2,2,6,6-tetramethyl-1-piperidinooxyl (i.e., 2,2,6,6-tetramethylpiperidinyl-1-oxy; TEMPO).

The amount of the radical generators is, for example, from about 0.0000001 to about 0.8 mole, preferably from about 0.0001 to about 0.7 mole, and more preferably from about 0.01 to about 0.6 mole, per 1 mole of the substrate.

### [Promoters]

Where necessary, one or more promoters (co-catalysts) can be used in combination with the nitrogen-containing cyclic compound (catalyst) in the production methods according to the present invention. Exemplary promoters include (i) compounds having a carbonyl group bound to an electron-withdrawing group, (ii) metallic compounds, (iii) organic salts each composed of a polyatomic cation or a polyatomic anion and its counter ion, which polyatomic cation or anion contains an element belonging to Group 15 or Group 16 of the Periodic Table and having at least one organic group bound thereto, and (iv) strong acids. Each of different promoters can be used alone or in combination.

In the compounds (i) having a carbonyl group bound to an electron-withdrawing group, exemplary electron-withdrawing groups to be bound to carbonyl group include fluoromethyl, trifluoromethyl, tetrafluoroethyl, phenyl, fluorophenyl, and pentafluorophenyl groups and other fluorine-substituted hydrocarbon groups. Examples of the compounds (i) include hexafluoroacetone, trifluoroacetic acid, pentafluorophenyl (methyl) ketone, pentafluorophenyl (trifluoromethyl) ketone, and benzoic acid. These compounds, if used, help to increase the reaction rate of a Baeyer-Villiger reaction, probably because they are converted into highly reactive peroxides in the system. The amount of the compounds (i) is from about 0.0001 to about 1 mole, and preferably from about 0.01 to about 0.7 mole, per 1 mole of the substrate.

Metallic elements constituting the metallic compounds (ii) are not especially limited and may be any of metallic elements belonging to Groups 1 to 15 of the Periodic Table. Exemplary metallic compounds (ii) include, of the metallic elements, inorganic compounds including elementary substances, hydroxides, oxides (including multicomponent oxides), halides (fluorides, chlorides, bromides, and iodides), salts of oxoacids (e.g., nitrates, sulfates, phosphates, borates, and carbonates), oxoacids, isopolyacids, and heteropolyacids; and organic compounds including salts of organic acids (e.g., acetates, propionates, prussiates (cyanides), naphthenates, and stearates), and complexes. Each of different metallic compounds (ii) can be used alone or in combination. The metallic compounds (ii), if used, may improve the selectivity of some reactions. The amount of the metallic compounds (ii) is, for example, from about 0.0001 to about 1 mole, and preferably from about 0.001 to about 0.5 mole, per 1 mole of the substrate.

In the organic salts (iii), exemplary elements belonging to Group 15 of the Periodic Table include N, P, As, Sb, and Bi; and exemplary elements belonging to Group 16 of the Periodic Table include O, S, Se, and Te. Among these elements, N, P, As, Sb, and S are preferred, of which N, P, and S are more preferred. Organic onium salts are preferred as the organic salts (iii). The amount of organic salts (iii) is, for example, from about 0.0001 to about 1 mole, and preferably from about 0.001 to about 0.5 mole, per 1 mole of the substrate.

Exemplary strong acids (iv) include mineral acids such as sulfuric acid, nitric acid, and hydrochloric acid; sulfonic acids such as p-toluenesulfonic acid; and strongly acidic cation-exchange resins. Each of different strong acids (iv) can be used alone or in combination. The amount of strong acids (iv) is, for example, from about 0.0001 to about 1 mole, and preferably from about 0.001 to about 0.5 mole, per 1 mole of the substrate.

### [Fluorine-Containing Alcohols]

The fluorine-containing alcohol are not especially limited and can be any of aliphatic alcohols and aromatic alcohols in which all or part of hydrogen atoms of their hydrocarbon group are substituted with fluorine atom(s). Such fluorine-containing alcohols can be whichever of monohydric alcohols and polyhydric alcohols.

Such fluorine-containing aliphatic alcohols include chain aliphatic alcohols and cyclic aliphatic alcohols. Preferred examples of chain aliphatic alcohols include fluorine-containing straight-chain aliphatic alcohols which are straight-chain alcohols having about 1 to about 20 carbon atoms, in which part or all of hydrogens of their hydrocarbon group are substituted with fluorine atom(s); and fluorine-containing branched-chain aliphatic alcohols which are branched-chain alcohols having about 3 to about 20 carbon atoms, in which part or all of hydrogens of their hydrocarbon group are substituted with fluorine atom(s). The hydrocarbon groups (or fluorinated hydrocarbon groups) in the fluorine-containing chain aliphatic alcohols may have one or more unsaturated bonds.

Specific examples of fluorine-containing straight-chain aliphatic alcohols in which part of hydrogens of the hydrocarbon group is substituted with fluorine atom(s) include 1,1-difluoroethanol, 1,1,2-trifluoroethanol, 2,2,2-trifluoroethanol, 1,1-difluoro-1-propanol, 1,2-difluoro-1-propanol, 1,2,3-trifluoro-1-propanol, 3,3,3-trifluoro-1-propanol, 1,1,2,2-tetrafluoro-1-propanol, 1,3-difluoro-1,3-propanediol, 2,3,4-trifluoro-2-butanol, 9,4,4-trifluoro-1-butanol, 3,3,4,4,4-pentafluoro-1-butanol, 1,1,2,2,3,3-hexafluoro-1-butanol, 1,1,2,2-tetrafluoro-1-butanol, 1,2,3,4-tetrafluoro-1-butanol, 3,3,4,4,4-pentafluoro-1-butanol, 1,2,3,4-tetrafluoro-1,4-butanediol, 1,1,2,2-tetrafluoro-1-pentanol, 5,5,5-trifluoro-2-pentanol, 4,4,5,5,5-pentafluoro-1-pentanol, 1,2,2,2-tetrafluoro-1-hexanol, and 5,5,6,6,6-pentafluoro-2-hexanol. Exemplary fluorine-containing branched-chain aliphatic alcohols include hexafluoroisopropanol, heptafluoroisopropanol, 3,3,3-trifluoro-2-trifluoromethyl-1-propanol, 2-trifluorornethyl-1-butanol, 2-trifluoromethyl-1,4-butanediol, and 2-trifluoromethyl-3,3,4,4,4-pentafluoro-1-butanol.

Examples of fluorine-containing cyclic aliphatic alcohols usable herein include alicyclic alcohols having about 3 to about 20 carbon atoms, such as cyclohexanol and cyclopentanol, and containing one or more fluorine atoms per molecule. The fluorine-containing cyclic aliphatic alcohols can contain fluorine atom(s) in any form not especially limited. Typically, fluorine atom(s) may be bound to ring-constituting carbon atom(s) or a fluorine-containing hydrocarbon group may be bound to the ring.

Examples of fluorine-containing aromatic alcohols usable herein include aromatic alcohols, such as benzyl alcohol and phenylethanol, which contain one or more fluorine atoms per molecule. They can contain fluorine atom(s) in any form not especially limited. Typically, a fluorinated hydrocarbon group may be substituted on their aromatic ring, or their chain hydrocarbon moiety may have fluorine atom(s). Representative examples of fluorine-containing aromatic alcohols include octafluorophenethyl alcohol.

Exemplary fluorine-containing alcohols further include heterogenous fluorine-containing alcohol resins such as fluorinated poly(vinyl alcohol)s.

Among them, fluorine-containing branched-chain aliphatic alcohols represented by following Formula (10) are preferably used as the fluorine-containing alcohols, of which hexafluoroisopropanol is especially preferably used:

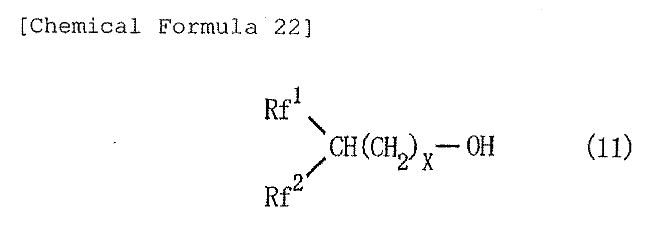

wherein Rf¹ and Rf² are the same as or different from each other and each represent a perfluoroalkyl group having 1 to 8 carbon atoms; and "x" is an integer of 0 to 8.

In addition, also preferably used herein are fluorine-containing straight-chain aliphatic alcohols having about 1 to about 15 carbon atoms, such as 1,1-difluoroethanol, 1,1,2-trifluoroethanol, and 2,2,2-trifluoroethanol; and fluorine-containing aromatic alcohols having about 7 to about 15 carbon atoms, such as octafluorophenethyl alcohol.

Fluorinated sulfonic acids can be used in the present invention instead of, or in combination with, fluorinated atom-containing alcohols. Exemplary fluorinated sulfonic acids include fluorinated alkanesulfonic acids such as trifluoromethanesulfonic acid, of which perfluoroalkanesulfonic acids are preferred; and strongly acidic ion-exchange resins such as fluorinated sulfonic acid polymers [perfluorosulfonic acid polymers (e.g., trade name: Nafion, supplied by Du Pont)]. Among them, perfluorosulfonic acid polymers are preferably used for their high conductivity to cation, chemical stability, and durability.

Though not especially limited, the amount (total amount) of at least one of fluorine-containing alcohols and fluorinated sulfonic acids can be chosen within broad ranges of, for example, 0.001 mole or more, preferably 0.05 mole or more, and more preferably 0.5 mole or more, per 1 mole of the secondary alcohol represented by Formula (1), or per 1 mole (total amount) of the peroxide represented by Formulae (3a) and (3b), or per 1 mole of the ketone represented by Formula (4). The fluorine-containing alcohols and/or fluorinated sulfonic acids can be used in large excess to the reaction raw material. It is also preferred to use the fluorine-containing alcohols and/or fluorinated sulfonic acids as solvents in the reaction. Each of different fluorine-containing alcohols and different fluorinated sulfonic acids can be used alone or in combination, respectively.

### [First Production Method of Esters or Lactones]

In the first production method of esters or lactones according to the present invention, a secondary alcohol represented by Formula (1) is oxidized by molecular oxygen in the presence of a nitrogen-containing cyclic compound containing a skeleton represented by Formula (I) as a constituent of its ring and at least one of a fluorine-containing alcohol and a fluorinated sulfonic acid to give an ester or lactone represented by Formula (2). This method is very useful as an industrial method, because a corresponding ester or lactone can be produced from a secondary alcohol in one step.

The oxidation reaction is performed in the presence of, or in the absence of, a solvent. Fluorine-containing alcohols can be used as solvents, as described above. Other examples of solvents usable herein include organic acids such as acetic acid and propionic acid; nitriles such as acetonitrile, propionitrile, and benzonitrile; amides such as formamide, acetamide, dimethylformamide (DMF), and dimethylacetamide; aliphatic hydrocarbons such as hexane and octane; halogenated hydrocarbons such as chloroform, dichloromethane, dichloroethane, carbon tetrachloride, chlorobenzene, and trifluoromethylbenzene; nitro compounds such as nitrobenzene, nitromethane, and nitroethane; esters such as ethyl acetate and butyl acetate; and mixtures of these solvents.

The reaction temperature can be chosen within ranges of, for example, from about 0°C to about 150°C, preferably from about 20°C to about 120°C, and more preferably from about 40°C to about 80°C, according typically to the type of the substrate. The reaction can be performed under normal atmospheric pressure or under a pressure (under a load).
The reaction time can be suitably chosen within ranges of, for example, from about 30 minutes to about 48 hours, according to the temperature and pressure of the reaction.

The reaction can be performed in the presence of, or under the flow of, molecular oxygen according to a common system such as a batch system, semi-batch system, or continuous system. The reaction converts the substrate secondary alcohol into a corresponding ester or lactone.

After the completion of the reaction, a reaction product, unreacted substrate and ketone, nitrogen-containing cyclic compound, fluorine-containing alcohol and/or fluorinated sulfonic acid, and solvent can be separated, purified, and recovered through a separation procedure such as filtration, concentration, distillation, extraction, crystallization, recrystallization, adsorption, or column chromatography, or through any combination of such separation procedures. The unreacted substrate and ketone, nitrogen-containing cyclic compound, fluorine-containing alcohol and/or fluorinated sulfonic acid, and solvent can each be recycled and reused to the oxidation step, after the separation by the separation procedure. The target compound can be produced with a high production efficiency by recycling and reusing the components such as unreacted substrate and ketone, nitrogen-containing cyclic compound, fluorine-containing alcohol and/or fluorinated sulfonic acid, and solvent.

At least one compound selected from indium compounds, selenium compounds, tellurium compounds, and polonium compounds may be added to the reaction system, or the reaction product after the oxidation reaction may be treated with at least one of these compounds. The addition of these compounds or the treatment with these compounds may improve the yield and/or selectivity of the target compound. Exemplary indium compounds include inorganic indium compounds such as indium trichloride; and indium complexes. Exemplary selenium compounds include inorganic selenium compounds such as selenium oxides (SeO₂ and SeO₃), selenium halides, and selenium oxyhalides; and organic selenium compounds. Exemplary tellurium compounds include inorganic tellurium compounds such as tellurium oxides (TheO₂ and TeO₃), tellurium halides or tellurium oxyhalides; and organic tellurium compounds. Exemplary polonium compounds include inorganic polonium compounds such as polonium oxides (PoO, PoO₂, and PoO₃), and polonium halides; and organic polonium compounds.

The amount of at least one compound selected from indium compounds, selenium compounds, tellurium compounds, and polonium compounds is, for example, from about 0.0001 to about 1 mole, preferably from about 0.001 to about 0.1 mole, and more preferably from about 0.005 to about 0.05 mole, per 1 mole of the substrate.

When the reaction product after oxidation reaction is treated with the compound, the treatment is performed in the presence of, or in the absence of, a solvent. Examples of the solvent include a variety of solvents for use in the oxidation reaction. The treatment may be performed by adding the above-mentioned compound(s) to the reaction mixture after oxidation reaction. The treatment is performed at a temperature of, for example, from about 0°C to about 150°C, preferably from about 20°C to about 120°C, and more preferably from about 40°C to about 80°C. The treatment may be performed under normal atmospheric pressure or under a pressure (under a load). The reaction time (treatment time) is, for example, from about 0.5 to about 24 hours, preferably from about 2 to about 10 hours, and more preferably from about 3 to about 8 hours.

### [Second Production Method of Esters or Lactones]

In the second production method of esters or lactones according to the present invention, a secondary alcohol represented by Formula (1) is oxidized by molecular oxygen in the presence of a nitrogen-containing cyclic compound containing a skeleton represented by Formula (I) as a constituent of its ring (this oxidation step is also referred to as "first step"); and is then treated with at least one of a fluorine-containing alcohol and a fluorinated sulfonic acid (this treating step is also referred to as "second step"), to give an ester or lactone represented by Formula (2).

The oxidation reaction in the first step is performed in the presence of, or in the absence of, a solvent. Exemplary solvents include organic acids such as acetic acid and propionic acid; nitriles such as acetonitrile, propionitrile, and benzonitrile; amides such as formamide, acetamide, dimethylformamide (DMF), and dimethylacetamide; aliphatic hydrocarbons such as hexane and octane; halogenated hydrocarbons such as chloroform, dichloromethane, dichloroethane, carbon tetrachloride, chlorobenzene, and trifluoromethylbenzene; nitro compounds such as nitrobenzene, nitromethane, and nitroethane; esters such as ethyl acetate and butyl acetate; and mixtures of these solvents. Among them, examples of often used solvents are organic acids such as acetic acid; nitriles such as acetonitrile and benzonitrile; halogenated hydrocarbons such as trifluoromethylbenzene; and esters such as ethyl acetate.

The reaction temperature can be chosen within broad ranges of, for example, from about 0°C to about 150°C, preferably from about 20°C to about 120°C, and more preferably from about 40°C to about 80°C, according typically to the type of the substrate. The reaction can be performed under normal atmospheric pressure or under a pressure (under a lead). The reaction time can be suitably chosen within ranges of, for example, from about 30 minutes to about 48 hours, according to the temperature and pressure of the reaction.

The reaction can be performed in the presence of, or under the flow of, molecular oxygen according to a common system such as a batch system, semi-batch system, or continuous system. Where necessary, the reaction product after the completion of the reaction is subjected to a suitable treatment such as concentration, dilution, solvent exchange, or purification, before being subjected to the second step.

As a result of the first step, the substrate secondary alcohol is oxidized to mainly give peroxides such as compounds represented by Formula (3a) and/or (3b). The secondary alcohol can also be converted into a corresponding hydroperoxide and/or ketone in this step.

In the second step, the reaction product after the oxidation reaction is treated with at least one of a fluorine-containing alcohol and a fluorinated sulfonic acid to give an ester or lactone represented by Formula (2).

The treatment with at least one of a fluorine-containing alcohol and a fluorinated sulfonic acid is performed in the presence of, or in the absence of, a solvent. Exemplary solvents herein include a variety of solvents for use in the first step. The solvent used in the first step may be the same as or different from one used in the second step. The treatment temperature is, for example, from about 0°C to about 150°C, preferably from about 20°C to about 120°C, and more preferably from about 40°C to about 80°C. The treatment may be performed under normal atmospheric pressure or under a pressure (under a load). The reaction time is, for example, from about 0.5 to about 24 hours, preferably from about 2 to about 10 hours, and more preferably from about 3 to about 8 hours.

As a result of the second step, an oxidation reaction product produced in the first step [for example, a peroxide represented by Formula (3a) and/or (3b)] is immediately decomposed and converted into a target corresponding ester or lactone.

After the completion of the first step and/or second step, the reaction product, unreacted substrate and ketone, nitrogen-containing cyclic compound, fluorine-containing alcohols and/or fluorinated sulfonic acid, and solvent can be separated, purified, and recovered, respectively, according to a separation procedure such as filtration, concentration, distillation, extraction, crystallization, recrystallization, adsorption, or column chromatography, or any combination of such separation procedures. After the separation by the separation procedure, the unreacted substrate and ketone, nitrogen-containing cyclic compound, fluorine-containing alcohol and/or fluorinated sulfonic acid, and solvent can also be recycled and reused in the first step and/or second step. The recycling of these components such as unreacted substrate and ketone, nitrogen-containing cyclic compound, fluorine-containing alcohol and/or fluorinated sulfonic acid, and solvent enables production of the target compound with a high production efficiency.

At least one compound selected from indium compounds, selenium compounds, tellurium compounds, and polonium compounds may be added to the reaction system of the first step or second step, or the reaction product after the second step may be treated with at least one of these compounds. The addition of these compounds or the treatment with these compounds may improve the yield and/or selectivity of the target compound. Examples of the indium compounds, selenium compounds, tellurium compounds, and polonium compounds usable herein and the amount thereof are as above. Conditions for the treatment of the reaction product of the second step with the compound, if performed, are as in the first production method of esters or lactones.

### [Third Production Method of Esters or Lactones]

In the third production method of esters or lactones according to the present invention, a peroxide represented by Formula (3a) and/or (3b) is treated with at least one of a fluorine-containing alcohol and a fluorinated sulfonic acid, to give an ester or lactone represented by Formula (2). The substituents R^{a}s and R^{b}s in Formulae (3a) and (3b) are as defined above.

The compounds represented by Formula (3a) and (3b) are formed in the first step of the second production method of esters or lactones, as described above.

A compound represented by Formula (3a) can be prepared by reacting a ketone represented by Formula (9) with hydrogen peroxide or a peroxide. In this case, the reaction can be performed in the presence of, or in the absence of, a solvent. The reaction temperature is, for example, from about 0°C to about 80°C, and preferably from about 10°C to about 40°C. After the completion of the reaction, the reaction product can be separated, purified, and recovered according to a separation procedure such as filtration, concentration, distillation, extraction, crystallization, recrystallization, adsorption, or column chromatography, or any combination of such separation procedures.

A compound represented by Formula (3b) can be prepared by reacting a ketone represented by Formula (9) with hydrogen peroxide or a peroxide in the presence of an acid such as hydrochloric acid. In this case, the reaction can be performed in the presence of, or in the absence of, a solvent. The reaction temperature is, for example, from about 0°C to about 80°C, and preferably from about 10°C to about 40°C. After the completion of the reaction, the reaction product can be separated, purified, and recovered according to a separation procedure such as filtration, concentration, distillation, extraction, crystallization, recrystallization, adsorption, or column chromatography, or any combination of such separation procedures.

The treatment of the peroxide represented by Formula (3a) and/or (3b) with at least one of a fluorine-containing alcohol and a fluorinated sulfonic acid is performed in the presence of, or in the absence of, a solvent. Exemplary solvents include a variety of solvents for use in the first step of the second production method of esters or lactones. The treatment temperature is, for example from about 0°C to about 150°C, preferably from about 10°C to about 120°C, and more preferably from about 15°C to about 80°C. The treatment may be performed under normal atmospheric pressure or under a pressure (under a load). The reaction time is, for example, from about 0.5 to about 24 hours, preferably from about 2 to about 10 hours, and more preferably from about 3 to about 8 hours.

As a result of the treatment with at least one of a fluorine-containing alcohol and a fluorinated sulfonic acid, the peroxide represented by Formula (3a) and/or (3b) is decomposed and converted into a target corresponding ester or lactone represented by Formula (2).

After the completion of the reaction, components such as the reaction product, unreacted raw material, fluorine-containing alcohol, and solvent can be separated, purified, and recovered, respectively, according to a separation procedure such as filtration, concentration, distillation, extraction, crystallization, recrystallization, adsorption, or column chromatography, or any combination of such separation procedures. After the separation by the separation procedure, the unreacted raw material, fluorine-containing alcohol and/or fluorinated sulfonic acid, and solvent can also be recycled to the reaction step. The recycling and reusing of these components enables production of the target compound with a high production efficiency.

At least one compound selected from indium compounds, selenium compounds, tellurium compounds, and polonium compounds may be added to the reaction system, or the reaction product after the treatment with a fluorine-containing alcohol may be further treated with any of these compounds. The addition of these compounds or the treatment with these compounds may improve the yield and/or selectivity of the target compound. Examples of the indium compounds, selenium compounds, tellurium compounds, and polonium compounds usable herein and the amount thereof are as above. When the reaction product after the treatment with a fluorine-containing alcohol is further treated with the compound, conditions for the further treatment are as in the first production method of esters or lactones.

### [Fourth Production Method of Esters or Lactones]

In the fourth production method of esters or lactones according to the present invention, a ketone represented by Formula (4) is oxidized by molecular oxygen in the presence of a secondary alcohol represented by Formula (5), a nitrogen-containing cyclic compound containing a skeleton represented by Formula (I) as a constituent of its ring, and at least one of a fluorine-containing alcohol and a fluorinated sulfonic acid, to give an ester or lactone represented by Formula (6). This method is very useful as an industrial method, because a corresponding ester or lactone can be produced from a ketone in one step.

The oxidation reaction is performed in the presence of, or in the absence of, a solvent. Exemplary solvents include a variety of solvents for use in the first production method of esters or lactones. The reaction temperature can be chosen within ranges of, for example, from about 0°C to about 150°C, preferably from about 20°C to about 120°C, and more preferably from about 40°C to about 80°C. The reaction may be performed under normal atmospheric pressure or under a pressure (under a load). The reaction time can be suitably chosen within ranges of, for example, about 30 minutes to about 48 hours, according to the temperature and pressure of the reaction.

The reaction can be performed in the presence of, or under the flow of, molecular oxygen according to a common system such as a batch system, semi-batch system, or continuous system. The reaction allows the substrate ketone to be converted into a corresponding ester or lactone.

After the completion of the reaction, the reaction product, unreacted substrate and secondary alcohol, nitrogen-containing cyclic compound, fluorine-containing alcohol and/or fluorinated sulfonic acid, and solvent can be separated, purified, and recovered, respectively, according to a separation procedure such as filtration, concentration, distillation, extraction, crystallization, recrystallization, adsorption, or column chromatography, or any combination of such separation procedures. After the separation by the separation procedure, the unreacted substrate and secondary alcohol, nitrogen-containing cyclic compound, fluorine-containing alcohol and/or fluorinated sulfonic acid, and solvent can be recycled to the oxidation step. The target compound can be produced with a high production efficiency by recycling and reusing components such as the unreacted substrate and secondary alcohol, nitrogen-containing cyclic compound, fluorine-containing alcohol and/or fluorinated sulfonic acid, and solvent.

At least one compound selected from indium compounds, selenium compounds, tellurium compounds, and polonium compounds may be added to the reaction system, or the reaction product after the oxidation reaction may be treated with any of these compounds. The addition of these compounds or the treatment with these compounds may improve the yield and/or selectivity of the target compound. Examples of the indium compounds, selenium compounds, tellurium compounds, and polonium compounds usable herein and the amount thereof are as above.

### [Fifth Production Method of Esters or Lactones]

In the fifth production method of esters or lactones according to the present invention, a ketone represented by Formula (4) is oxidized by molecular oxygen in the presence of a secondary alcohol represented by Formula (5) and a nitrogen-containing cyclic compound containing a skeleton represented by Formula (I) as a constituent of its ring (this oxidation step is also referred to as "first step"), and is then treated with at least one of a fluorine-containing alcohol and a fluorinated sulfonic acid (this treating step is also referred to as "second step"), to give an ester or lactone represented by Formula (6).

The oxidation reaction in the first step is performed in the presence of, or in the absence of, a solvent. Exemplary solvents include organic acids such as acetic acid and propionic acid; nitriles such as acetonitrile, propionitrile, and benzonitrile; amides such as formamide, acetamide, dimethylformamide (DMF), and dimethylacetamide; aliphatic hydrocarbons such as hexane and octane; halogenated hydrocarbons such as chloroform, dichloromethane, dichloroethane, carbon tetrachloride, chlorobenzene, and trifluoromethylbenzene; nitro compounds such as nitrobenzene, nitromethane, and nitroethane; esters such as ethyl acetate and butyl acetate; and mixtures of these solvents. Examples of often used solvents are organic acids such as acetic acid; nitriles such as acetonitrile and benzonitrile; halogenated hydrocarbons such as trifluoromethylbenzene; and esters such as ethyl acetate.

The reaction temperature can be chosen within ranges of, for example, from about 0°C to about 150°C, preferably from about 20°C to about 120°C, and more preferably from about 40°C to about 80°C, according typically to the type of the substrate. The reaction can be performed under normal atmospheric pressure or under a pressure (under a load). The reaction time can be suitably chosen within ranges of, for example, about 30 minutes to about 48 hours, according to the temperature and pressure of the reaction.

The reaction can be performed in the presence of, or under the flow of, molecular oxygen according to a common system such as a batch system, semi-batch system, or continuous system. Where necessary, the reaction product after the completion of the reaction is subjected to a suitable treatment such as concentration, dilution, solvent exchange, or purification, before being subjected to the second step.

It is speculated that the secondary alcohol represented by Formula (5) is oxidized as a result of the first step and mainly gives peroxides such as compounds represented by following Formula (10a) and/or (10b): 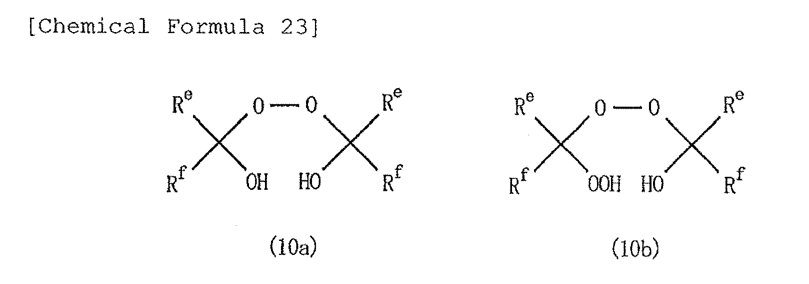 wherein R^{e}s and R^{f}s are as defined above.

In the second step, the reaction product after the oxidation reaction is treated with at least one of a fluorine-containing alcohol and a fluorinated sulfonic acid, to give an ester or lactone represented by Formula (6).

The treatment with at least one of a fluorine-containing alcohol and a fluorinated sulfonic acid is performed in the presence of, or in the absence of, a solvent. Exemplary solvents include a variety of solvents for use in the first step. The solvent used in the first step may be the same as or different from one used in the second step. The treatment temperature is, for example, from about 0°C to about 150°C, preferably from about 20°C to about 120°C, and more preferably from about 40°C to about 80°C. The treatment may be performed under normal atmospheric pressure or under a pressure (under a load). The reaction time is, for example, from about 0.5 to about 24 hours, preferably from about 2 to about 10 hours, and more preferably from about 3 to about 8 hours.

It is speculated that the oxidation reaction product [for example, compounds represented by Formula (10a) and (10b)] formed in the first step reacts with the ketone represented by Formula (4) to give a target ester or lactone represented by Formula (6) [ester or lactone corresponding to the ketone represented by Formula (4)] in the second step.

After the completion of the first step and/or second step, the reaction product, unreacted substrate and secondary alcohol, nitrogen-containing cyclic compound, fluorine-containing alcohol and/or fluorinated sulfonic acid, and solvent can be separated, purified, and recovered, respectively, according to a separation procedure such as filtration, concentration, distillation, extraction, crystallization, recrystallization, adsorption, or column chromatography, or any combination of such separation procedures. After the separation by the separation procedure, the unreacted substrate and secondary alcohol, nitrogen-containing cyclic compound, fluorine-containing alcohol and/or fluorinated sulfonic acid, and solvent can also be recycled to the first step and/or second step. The target compound can be produced with a high production efficiency by recycling and reusing the components such as the unreacted substrate and secondary alcohol, nitrogen-containing cyclic compound, fluorine-containing alcohol and/or fluorinated sulfonic acid, and solvent.

At least one compound selected from indium compounds, selenium compounds, tellurium compounds, and polonium compounds may be added to the reaction system of the first step or second step, or the reaction product after the second step may be treated with any of these compounds. The addition of these compounds or the treatment with these compounds may improve the yield and/or selectivity of the target compound. Examples of the indium compounds, selenium compounds, tellurium compounds, and polonium compounds usable herein and the amount thereof are as above. Conditions for the treatment of the reaction product after the second step with the compound, if performed, are as in the first production method of esters or lactones.

The first, second, and third production methods of esters or lactones according to the present invention enable direct production of an ester or lactone in a high selectivity from a secondary alcohol or an oxidation product thereof (peroxide). According to the production methods of the present invention, an aliphatic secondary alcohol, if used as the substrate, gives a corresponding ester; and an alicyclic alcohol, if used as the substrate, gives a corresponding lactone having one more member than that of the alicyclic alcohol. In the latter case, a corresponding alicyclic carboxylic acid having one less member than that of the raw material alicyclic alcohol may be formed as a byproduct. The production methods are useful as methods for directly producing corresponding lactones (e.g., ε-caprolactones) by oxidizing cycloalkanols (cycloalkanols such as cyclohexanols) having 3 to 20 members, which may have substituent(s) such as alkyl group(s), and alicyclic alcohols corresponding to bridged hydrocarbon rings having about two to about four rings.

The fourth and fifth production methods of esters or lactones according to the present invention enable direct production of an ester or lactone in a high selectivity from a ketone. According to the production methods according to the present invention, an open-chain ketone, if used, gives a corresponding ester; and a cyclic ketone, if used, gives a corresponding lactone having one more member than that of the material cyclic ketone. These production methods are useful especially as methods for producing corresponding lactones (e.g., ε-caprolactones) by oxidizing cycloalkanones (cycloalkanones such as cyclohexanones) having 3 to 20 members, which may have substituent(s) such as alkyl group(s), and cyclic ketone corresponding to bridged hydrocarbon rings having about two to about four rings.

The resulting esters and lactones are usable as pharmaceuticals, flavors, dyestuffs, intermediates for organic syntheses, and raw materials for polymeric resins.

### EXAMPLES

The present invention will be illustrated in further detail with reference to several examples below. It should be noted, however, these examples are never construed to limit the scope of the present invention.

### EXAMPLE 1

A mixture of 10 millimoles (mmol) of cyclohexanol, 40 mmol of cyclohexanone, 6 mmol of N-hydroxyphthalimide, 3 mmol of azobisisobutyronitrile (AIBN), and 30 mL of hexafluoroisopropanol was stirred at 60°C in an oxygen atmosphere (at 1 atmosphere, i.e., 0.1. MPa) for 20 hours. The reaction mixture was analyzed by gas chromatography and found to contain 3.3 mmol of ε-caprolactone, 31 mmol of cyclohexanol, and 14 mmol of cyclohexanone. The selectivity for ε-caprolactone was 66% on the basis of total conversion from cyclohexanol and cyclohexanone.

### EXAMPLE 2

A mixture of 30 mmol of cyclohexanol, 60 mmol of cyclohexanone, 6 mmol of N-hydroxyphthalimide, 3 mmol of azobisisobutyronitrile (AIBN), and 30 mL of hexafluoroisopropanol was stirred at 60°C in an oxygen atmosphere (at 1 atmosphere, i.e., 0.1 MPa) for 20 hours. The reaction mixture was analyzed by gas chromatography and found to contain 9.6 mmol of ε-caprolactone, 35 mmol of cyclohexanol, and 26 mmol of cyclohexanone. The selectivity for ε-caprolactone was 33% on the basis of total conversion from cyclohexanol and cyclohexanone.

### EXAMPLE 3

A mixture of 60 mmol of cyclohexanol, 120 mmol of cyclohexanone, 6 mmol of N-hydroxyphthalimide, 3 mmol of azobisisobutyronitrile (AIBN), and 30 mL of hexafluoroisopropanol was stirred at 60°C in an oxygen atmosphere (at 1 atmosphere, i.e., 0.1 MPa) for 20 hours. The reaction mixture was analyzed by gas chromatography and found to contain 17 mmol of ε-caprolactone, 41 mmol of cyclohexanol, and 72 mmol of cyclohexanone. The selectivity for ε-caprolactone was 25% on the basis of total conversion from cyclohexanol and cyclohexanone.

### EXAMPLE 4

A mixture of 60 mmol of cyclohexanol, 120 mmol of cyclohexanone, 6 mmol of N-hydroxyphthalimide, 3 mmol of azobisisobutyronitrile (AIBN), and 30 mL of hexafluoroisopropanol was stirred at 60°C in an oxygen atmosphere (at 1 atmosphere, i.e., 0.1 MPa) for 30 hours. The reaction mixture was analyzed by gas chromatography and found to contain 24 mmol of ε-caprolactone, 64 mmol of cyclohexanol, and 71 mmol of cyclohexanone. The selectivity for ε-caprolactone was 53% on the basis of total conversion from cyclohexanol and cyclohexanone.

### EXAMPLE 5

A mixture of 60 mmol of cyclohexanol, 120 mmol of cyclohexanone, 6 mmol of N-hydroxyphthalimide, 3 mmol of azobisisobutyronitrile (AIBN), and 30 mL of hexafluoroisopropanol was stirred at 75°C in an oxygen atmosphere (at 1 atmosphere, i.e., 0.1 MPa) for 20 hours. The reaction mixture was analyzed by gas chromatography and found to contain 30 mmol of ε-caprolactone, 55 mmol of cyclohexanol, and 83 mmol of cyclohexanone. The selectivity for ε-caprolactone was 71% on the basis of total conversion from cyclohexanol and cyclohexanone.

### EXAMPLE 6

A mixture of 60 mmol of cyclohexanol, 120 mmol of cyclohexanone, 6 mmol of N-hydroxyphthalimide, 3 mmol of azobisisobutyronitrile (AIBN), and 30 mL of octafluorophenethyl alcohol was stirred at 90°C in an oxygen atmosphere (at 1 atmosphere, i.e., 0.1 MPa) for 20 hours. The reaction mixture was analyzed by gas chromatography and found to contain 45 mmol of ε-caprolactone, 40 mmol of cyclohexanol, and 81 mmol of cyclohexanone. The selectivity for ε-caprolactone was 76% on the basis of total conversion from cyclohexanol and cyclohexanone.

### EXAMPLE 7

A mixture of 60 mmol of cyclohexanol, 120 mmol of cyclohexanone, 6 mmol of N-hydroxyphthalimide, 3 mmol of azobisisobutyronitrile (AIBN), and 30 mL of hexafluoroisopropanol was placed in an autoclave and stirred at 90°C in the air under a pressure (2 MPa) for 20 hours. The reaction mixture was analyzed by gas chromatography and found to contain 41 mmol of ε-caprolactone, 42 mmol of cyclohexanol, and 82 mmol of cyclohexanone. The selectivity for ε-caprolactone was 73% on the basis of total conversion from cyclohexanol and cyclohexanone.

### EXAMPLE 8

A mixture of 60 mmol of cyclohexanol, 120 mmol of cyclohexanone, 6 mmol of N-hydroxyphthalimide, 3 mmol of azobisisobutyronitrile (AIBN), and 30 mL of trifluoroethanol was stirred at 75°C in an oxygen atmosphere (at 1 atmosphere, i.e., 0.1 MPa) for 20 hours. The reaction mixture was analyzed by gas chromatography and found to contain 26 mmol of ε-caprolactone, 33 mmol of cyclohexanol, and 89 mmol of cyclohexanone. The selectivity for ε-caprolactone was 45% on the basis of total conversion from cyclohexanol and cyclohexanone.

### EXAMPLE 9

A mixture of 60 mmol of cyclohexanol, 120 mmol of cyclohexanone, 6 mmol of N-hydroxyphthalimide, 3 mmol of azobisisobutyronitrile (AIBN), and 10 mL of ethyl acetate was stirred at 75°C in an oxygen atmosphere (at 1 atmosphere, i.e., 0.1 MPa) for 20 hours. Subsequently 30 mL of hexafluoroisopropanol was added, followed by stirring at the same temperature for 5 hours. The reaction mixture was analyzed by gas chromatography and found to contain 33 mmol of ε-caprolactone, 31 mmol of cyclohexanol, and 84 mmol of cyclohexanone. The selectivity for ε-caprolactone was 51% on the basis of total conversion from cyclohexanol and cyclohexanone.

### EXAMPLE 10

A mixture of 60 mmol of cyclohexanol, 120 mmol of cyclohexanone, 6 mmol of N-hydroxyphthalimide, 3 mmol of azobisisobutyronitrile (AIBN), and 30 mL of hexafluoroisopropanol was stirred at 75°C in an oxygen atmosphere (at 1 atmosphere, i.e., 0.1 MPa) for 20 hours. Subsequently 4.5 mmol of indium trichloride was added, followed by stirring at 25°C for 5 hours. The reaction mixture was analyzed by gas chromatography and found to contain 17 mmol of ε-caprolactone, 86 mmol of cyclohexanol, and 74 mmol of cyclohexanone. The selectivity for ε-caprolactone was 85% on the basis of total conversion from cyclohexanol and cyclohexanone.

### EXAMPLE 11

A mixture of 60 mmol of cyclohexanol, 120 mmol of cyclohexanone, 6 mmol of N-hydroxyphthalimide, and 3 mmol of azobisisobutyronitrile (AIN) was stirred at 75°C in an oxygen atmosphere (at 1 atmosphere, i.e., 0.1 MPa) for 20 hours. Subsequently 5 g of a fluorinated sulfonic acid polymer (trade name: Nafion, supplied by Du Pont) was added, followed by stirring at 60°C for 2 hours. The reaction mixture was analyzed by gas chromatography and found to contain 13 mmol of ε-caprolactone, 31 mmol of cyclohexanol, and 103 mmol of cyclohexanone.

### EXAMPLE 12

In a 500-mL reactor was placed 300 mL of cyclohexanone; and 50 mL of a 35-percent by weight hydrogen peroxide solution was added dropwise thereto while maintaining the mixture at room temperature, followed by carrying out a reaction for 3 hours. The resulting reaction mixture was extracted with ethyl acetate, washed with water, from which ethyl acetate was distilled off under reduced pressure, to give a white solid. The solid was recrystallized from methanol and thereby yielded 1,1'-dihydroxydicyclohexyl peroxide represented by following Formula (12): 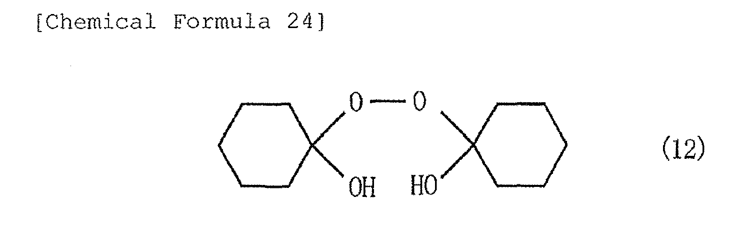 In 30 mL of hexafluoroisopropanol was dissolved 5 mmol of 1,1'-dihydroxydicyclohexyl peroxide, followed by stirring at 25°C for 4 hours. The reaction mixture was analyzed by gas chromatography and found to contain 4.8 mmol of ε-caprolactone and 5.2 mmol of cyclohexanone.

### EXAMPLE 13

In a 500-mL reactor were placed 2 moles of cyclohexanone and 20 mL of 2 M HCl; and 2 moles of a 35-percent by weight hydrogen peroxide solution was added dropwise thereto while maintaining the mixture at room temperature, followed by carrying out a reaction for 3 hours. The resulting reaction mixture was extracted with ethyl acetate, washed with water, from which ethyl acetate was distilled off under reduced pressure, to give a white solid. The solid was recrystallized from acetic acid and thereby yielded 1-hydroxy-1'-hydroperoxydicyclohexyl peroxide represented by following Formula (13): 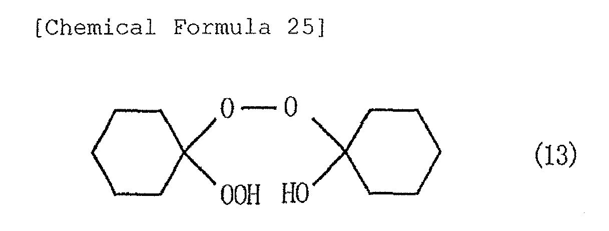 In 30 mL of hexafluoroisopropanol was dissolved 5 mmol of 1-hydroxy-1'-hydroperoxydicyclohexyl peroxide, followed by stirring at 60°C for 4 hours. The reaction mixture was analyzed by gas chromatography and found to contain 7 mmol of ε-caprolactone and 2.9 mmol of cyclohexanone.

### EXAMPLE 14

A mixture of 6 mmol of benzhydrol, 2 mmol of cyclohexanone, 0.3 mmol of N-hydraxyphthalimide, 0.15 mmol of azobisisobutyronitrile (AIBN), and 3 mL of acetonitrile was stirred at 75°C in an oxygen atmosphere (at 1 atmosphere, i.e., 0.1 MPa) for 20 hours. Subsequently the solvent was removed, and 6 mL of hexafluoroisopropanol was added, followed by stirring at 60°C for 24 hours. The reaction mixture was analyzed by gas chromatography and found that there was produced ε-caprolactone in a yield of 52% on the basis of cyclohexanone.

### EXAMPLE 15

A mixture of 6 mmol of benzhydrol, 2 mmol of cyclohexanone, 0.3 mmol of N-hydroxyphthalimide, 0.15 mmol of azobisisobutyronitrile (AIBN), and 3 mL of acetonitrile was stirred at 75°C in an oxygen atmosphere (at 1 atmosphere, i.e., 0.1 MPa) for 20 hours. Subsequently the solvent was removed, and 6 mL of hexafluoroisopropanol and 0.02 mmol of p-toluenesulfonic acid were added, followed by stirring at 60°C for 24 hours. The reaction mixture was analyzed by gas chromatography and found that there was produced ε-caprolactone in a yield of 64% on the basis of cyclohexanone.

### EXAMPLE 16

A mixture of 6 mmol of benzhydrol, 2 mmol of cyclopentadecanone, 0.3 mmol of N-hydroxyphthalimide, 0.15 mmol of azobisisobutyronitrile (AIBN), and 3 mL of acetonitrile was stirred at 75°C in an oxygen atmosphere (at 1 atmosphere, i.e., 0.1 MPa) for 20 hours. Subsequently the solvent was removed, and 6 mL of hexafluoroisopropanol and 0.02 mmol of p-toluenesulfonic acid were added, followed by stirring at 60°C for 24 hours. The reaction mixture was analyzed by gas chromatography and found that there was produced cyclopentadecanolide in a yield of 58% on the basis of cyclopentadecanone.

### EXAMPLE 17

In a 500-mL reactor was placed 300 mL of cyclohexanone; and 50 mL of a 35 percent by weight-hydrogen peroxide solution was added dropwise thereto while maintaining the mixture at room temperature, followed by carrying out a reaction for 3 hours. The resulting reaction mixture was extracted with ethyl acetate, washed with water, from which ethyl acetate was distilled off under reduced pressure, to give a white solid. The solid was recrystallized from methanol and thereby yielded 1,1'-dihydroxydicyclohexyl peroxide represented by Formula (12). Next, 5 mmol of 1,1'-dihydroxydicyclohexyl peroxide was dissolved in 30 mL of heptane, and 0.1 g of a fluorinated sulfonic acid polymer (trade name: Nafion, supplied by Du Pont) was added thereto, followed by stirring at 25°C for 4 hours. The reaction mixture was analyzed by gas chromatography and found to contain 2.1 mmol of ε-caprolactone.

### EXAMPLE 18

A mixture of 6 mmol of benzhydrol, 2 mmol of cyclopentadecanone, 0.3 mmol of N-hydroxyphthalimide, 0.15 mmol of azobisisobutyronitrile (AIBN), and 3 mL of acetonitrile was stirred at 75°C in an oxygen atmosphere (at 1 atmosphere, i.e., 0.1 MPa) for 22 hours. Subsequently the solvent was removed, and 6 mL of hexafluoroisopropanol was added, followed by stirring at 60°C for 24 hours. The reaction mixture was analyzed by gas chromatography and found that there were produced cyclopentadecanolide in a yield of 37% on the basis of cyclopentadecanone, and diphenyl ketone in a yield of 94% on the basis of benzhydrol. The conversions from cyclopentadecanone and from benzhydrol were 77% and 99%, respectively.

### EXAMPLE 19

A mixture of 6 mmol of benzhydrol, 2 mmol of cyclopentadecanone, 0.3 mmol of N-hydroxyphthalimide, 0.15 mmol of azobisisobutyronitrile (AIBN), and 3 mL of acetonitrile was stirred at 75°C in an oxygen atmosphere (at 1 atmosphere, i.e., 0.1 MPa) for 22 hours. Subsequently the solvent was removed, and 6 mL of hexafluoroisopropanol and 0.02 mmol of p-toluenesulfonic acid were added, followed by stirring at 60°C for 24 hours. The reaction mixture was analyzed by gas chromatography and found that there were produced cyclopentadecanolide in a yield of 52% on the basis of cyclopentadecanone, and diphenyl ketone in a yield of 94% on the basis of benzhydrol. The conversions from cyclopentadecanone and from benzhydrol were 91% and 99%, respectively.

### EXAMPLE 20

A mixture of 6 mmol of benzhydrol, 4 mmol of cyclopentadecanone, 0.3 mmol of N-hydroxyphthalimide, 0.15 mmol of azobisisobutyronitrile (AIBN), and 3 mL of acetonitrile was stirred at 75°C in an oxygen atmosphere (at 1 atmosphere, i.e., 0.1 MPa) for 22 hours. Subsequently the solvent was removed, and 6 mL of hexafluoroisopropanol was added, followed by stirring at 60°C for 24 hours. The reaction mixture was analyzed by gas chromatography and found that there were produced cyclopentadecanolide in a yield of 26% on the basis of cyclopentadecanone, and diphenyl ketone in a yield of 96% on the basis of benzhydrol. The conversions from cyclopentadecanone and from benzhydrol were 63% and 99%, respectively.

### Industrial Applicability

The present invention enables industrially efficient production of corresponding esters or lactones from secondary alcohols or oxidation products thereof (dimerized peroxides corresponding to the secondary alcohols) in a high selectivity through a simple and easy operation.

## Claims

1. A method for producing an ester or lactone, the method comprising the step of oxidizing a secondary alcohol by molecular oxygen in the presence of a nitrogen-containing cyclic compound and at least one of a fluorine-containing alcohol and a fluorinated sulfonic acid, the secondary alcohol represented by following Formula (1): 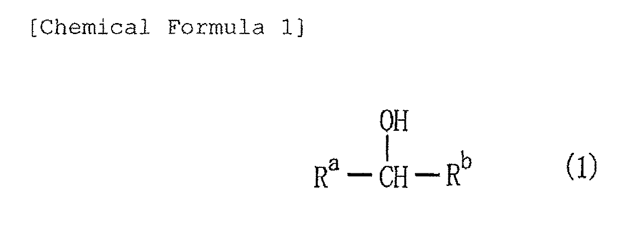 (wherein R^{a} and R^{b} are the same as or different from each other and each represent an organic group having a carbon atom at a bonding site with the adjacent carbon atom, or R^{a} and R^{b} may be combined to form a ring together with the adjacent carbon atom),
the nitrogen-containing cyclic compound containing, as a constituent of its ring, a skeleton represented by following Formula (I): 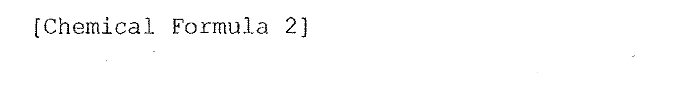 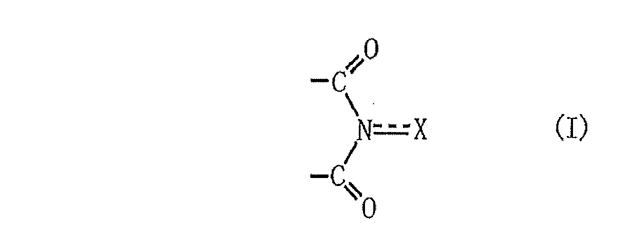 (wherein X represents an oxygen atom or an -OR group where R represents a hydrogen atom or a hydroxyl-protecting group), to give a compound represented by following Formula (2): 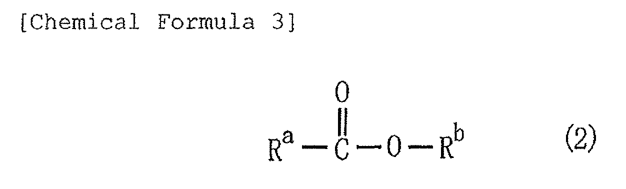 (wherein R^{a} and R^{b} are the same as or different from each other and each represent an organic group having a carbon atom at a bonding site with the adjacent carbonyl-carbon atom or oxygen atom, or R^{a} and R^{b} may be combined to form a ring together with the adjacent carbonyl-carbon atom and oxygen atom).

2. The method for producing an ester or lactone, according to claim 1, wherein oxidation of the secondary alcohol represented by Formula (1) is performed in the presence of a ketone.

3. A method for producing an ester or lactone, the method comprising the steps of: oxidizing a secondary alcohol by molecular oxygen in the presence of a nitrogen-containing cyclic compound, the secondary alcohol represented by following Formula (1): 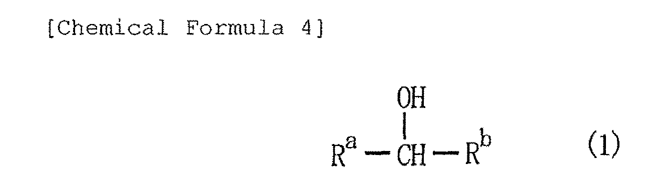 (wherein R^{a} and R^{b} are the same as or different from each other and each represent an organic group having a carbon atom at a bonding site with the adjacent carbon atom, or R^{a} and R^{b} may be combined to form a ring together with the adjacent carbon atom),
the nitrogen-containing cyclic compound containing, as a constituent of its ring, a skeleton represented by following Formula (I): 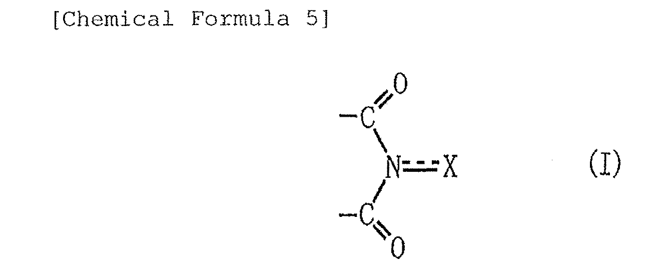 [wherein X represents an oxygen atom or an -OR group where R represents a hydrogen atom or a hydroxyl-protecting group]; and treating the oxidation product with at least one of a fluorine-containing alcohol and a fluorinated sulfonic acid, to give a compound represented by following Formula (2): 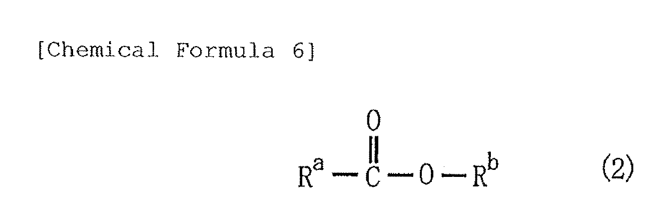 (wherein R^{a} and R^{b} are the same as or different from each other and each represent an organic group having a carbon atom at a bonding site with the adjacent carbonyl-carbon atom or oxygen atom, or R^{a} and R^{b} may be combined to form a ring together with the adjacent carbonyl-carbon atom and oxygen atom).

4. The method for producing an ester or lactone, according to claim 3, wherein oxidation of the secondary alcohol represented by Formula (1) is performed in the presence of a ketone.

5. A method for producing an ester or lactone, the method comprising the step of treating a peroxide with at least one of a fluorine-containing alcohol and a fluorinated sulfonic acid, the peroxide represented by following Formula (3a) and/or (3b): 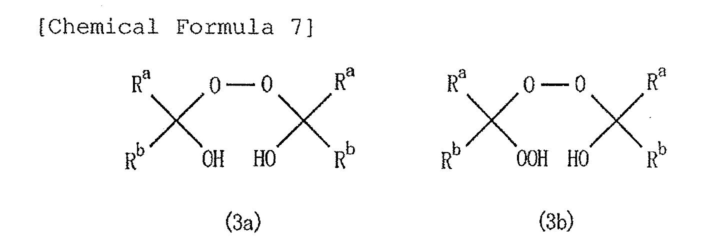 (wherein R^{a}s and R^{b}s are the same as or different from one another and each represent an organic group having a carbon atom at a bonding site with the adjacent carbon atom, or R^{a} and R^{b} may be combined to form a ring together with the adjacent carbon atom),
to give a compound represented by following Formula (2): 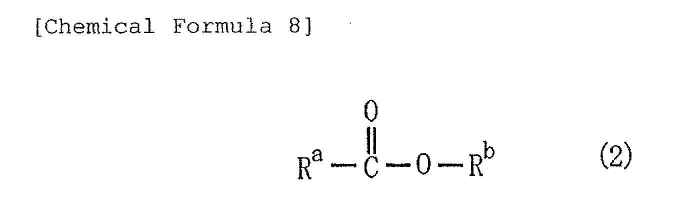 (wherein R^{a} and R^{b} are the same as or different from each other and each represent an organic group having a carbon atom at a bonding site with the adjacent carbonyl-carbon atom or oxygen atom, or R^{a} and R^{b} may be combined to form a ring together with the adjacent carbonyl-carbon atom and oxygen atom).

6. A method for producing an ester or lactone, the method comprising the step of oxidizing a ketone by molecular oxygen in the presence of a secondary alcohol, a nitrogen-containing cyclic compound, and at least one of a fluorine-containing alcohol and a fluorinated sulfonic acid, the ketone represented by following Formula (4): 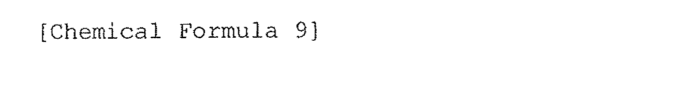 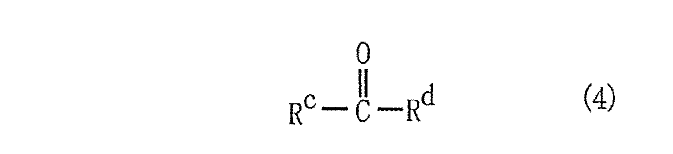 (wherein R^{c} and R^{d} are the same as or different from each other and each represent an organic group having a carbon atom at a bonding site with the adjacent carbon atom, or R^{c} and R^{d} may be combined to form a ring together with the adjacent carbon atom),
the secondary alcohol represented by following Formula (5): 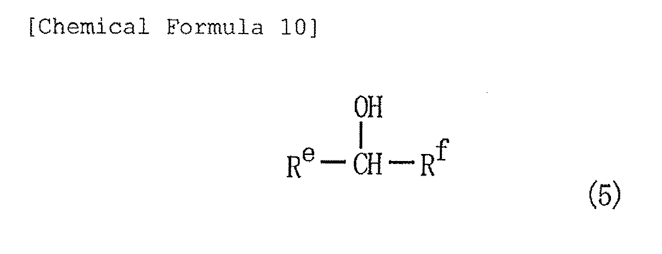 (wherein R^{e} and R^{f} are the same as or different from each other and each represent an organic group having a carbon atom at a bonding site with the adjacent carbon atom, and wherein R^{e} and R^{f} may be combined to form a ring together with the adjacent carbon atom),
the nitrogen-containing cyclic compound containing, as a constituent of its ring, a skeleton represented by following Formula (I): 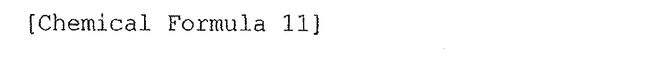 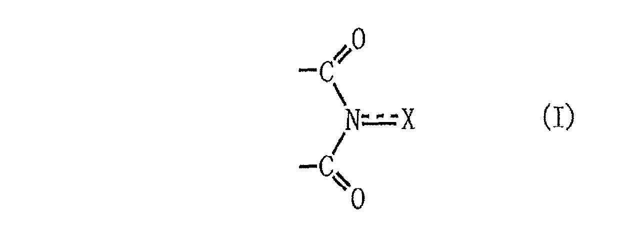 [wherein X represents an oxygen atom or an -OR group where R represents a hydrogen atom or a hydroxyl-protecting group], to give a compound represented by following Formula (6): 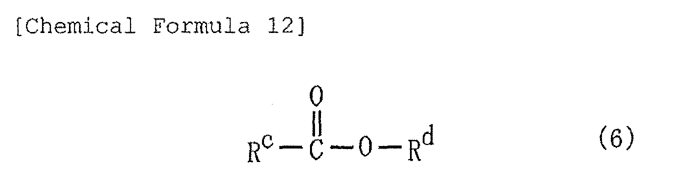 (wherein R^{c} and R^{d} are the same as or different from each other and each represent an organic group having a carbon atom at a bonding site with the adjacent carbonyl-carbon atom or oxygen atom, or R^{c} and R^{d} may be combined to form a ring together with the adjacent carbonyl-carbon atom and oxygen atom).

7. A method for producing an ester or lactone, the method comprising the steps of: oxidizing a ketone by molecular oxygen in the presence of a secondary alcohol and a nitrogen-containing cyclic compound, the ketone represented by following Formula (4): 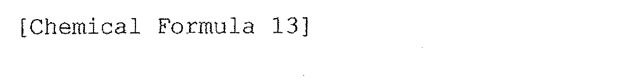 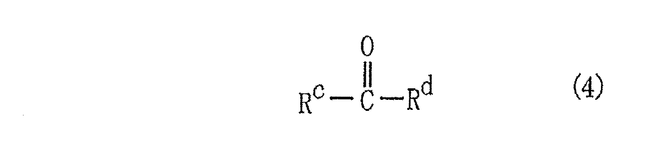 (wherein R^{c} and R^{d} are the same as or different from each other and each represent an organic group having a carbon atom at a bonding site with the adjacent carbon atom, or R^{c} and R^{d} may be combined to form a ring together with the adjacent carbon atom),
the secondary alcohol represented by following Formula (5): 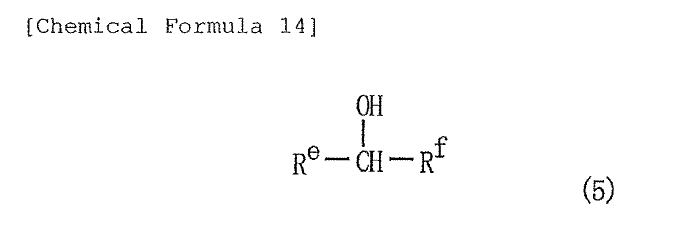 (wherein R^{e} and R^{f} are the same as or different from each other and each represent an organic group having a carbon atom at a bonding site with the adjacent carbon atom, and wherein R^{e} and R^{f} may be combined to form a ring together with the adjacent carbon atom),
the nitrogen-containing cyclic compound containing, as a constituent of its ring, a skeleton represented by following Formula (I): 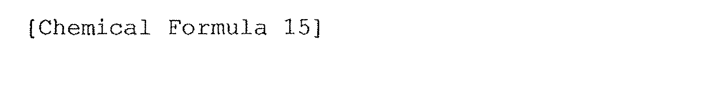 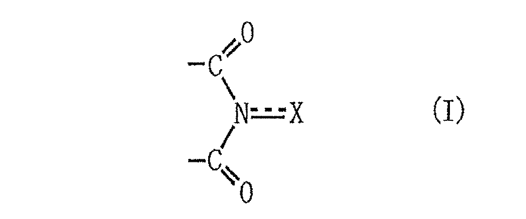 [wherein X represents an oxygen atom or an -OR group where R represents a hydrogen atom or a hydroxyl-protecting group]; and treating the oxidation product with at least one of a fluorine-containing alcohol and a fluorinated sulfonic acid, to give a compound represented by following Formula (6): 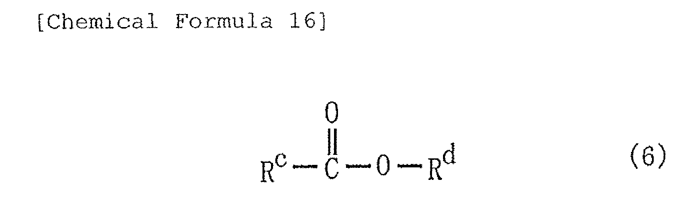 (wherein R^{c} and R^{d} are the same as or different from each other and each represent an organic group having a carbon atom at a bonding site with the adjacent carbonyl-carbon atom or oxygen atom, or R^{c} and R^{d} may be combined to form a ring together with the adjacent carbonyl-carbon atom and oxygen atom).
